# NEUE EUROPÄISCHE PATENTSCHRIFT

(11) **EP 2 558 116 B2**
(45) Veröffentlichungstag und Bekanntmachung des Hinweises auf die Entscheidung über den Einspruch: **08.01.2020**
(45) Hinweis auf die Patenterteilung: 22.03.2017
(21) Anmeldenummer: 11727394.6
(22) Anmeldetag: 15.04.2011
(51) Int. Cl.: A61K 39/00, A61K 39/12, A61K 35/761

(54) **MITTEL ZUR LOKALEN BEHANDLUNG VON ZERVIXDYSPLASIEN**
AGENT FOR LOCALLY TREATING CERVICAL DYSPLASIAS
AGENT DE TRAITEMENT LOCAL DE DYSPLASIES CERVICALES

(30) Priorität: 16.04.2010 DE 102010016475
(43) Veröffentlichungstag der Anmeldung: 20.02.2013
(73) Patentinhaber: General Vectors GmbH, 12203 Berlin (DE)
(72) Erfinder: CICHON, Günter, 12200 Berlin (DE)
(74) Vertreter: Hertin und Partner Rechts- und Patentanwälte PartG mbB
(86) Internationale Anmeldenummer: PCT/DE2011/075075
(87) Internationale Veröffentlichungsnummer: WO 2011/127924

(56) Entgegenhaltungen:
- WO-A1-2004/060408
- WO-A1-2007/121893
- WO-A1-2008/138648
- WO-A1-2009/106362
- WO-A2-2008/106646
- US-B2- 6 706 728
- US-B2- 7 659 071
- STEINWAERDER D S ET AL.: "HUMAN PAPILLOMA VIRUS E6 AND E7 PROTEINS SUPPORT DNA REPLICATION OFADENOVIRUSES DELETED FOR THE E1A AND E1B GENES", MOLECULAR THERAPY., Bd. 4, Nr. 3, September 2001 (2001-09), Seiten 211-216, XP001061267, in der Anmeldung erwähnt
- RUDIN C M ET AL: "An attenuated adenovirus ONYX-015 as mouthwash therapy for premalignant oral dysplasia", JOURNAL OF CLINICAL ONCOLOGY, Bd. 21, Nr. 24, 15. Dezember 2003 (2003-12-15), Seiten 4546-4552, XP002982638, AMERICAN SOCIETY OF CLINICAL ONCOLOGY, US ISSN: 0732-183X, DOI: 10.1200/JCO.2003.03.544
- HOFFMANN, CORINNA et al.: "Combining T- cell vaccination and application of agonistic anti-GITR mAb (DTA-1) induces complete eradication of HPV oncogene expressing tumors in mice", Journal of Immunotherapy, vol. 33, no. 2, February 2010 (2010-02), pages 136-145,
- DE SOUZA, APD. et al.: "Genital CD 8+T cell response to HIV-1 gag in mice immunized by mucosal routes with a recombinant simian adenovirus", Vaccine, vol. 25, no. 1, 2007, pages 109-116,
- LANZA, SILVIA R. et al.: "Simian recombinant adenovirus delivered by the mucosal route modulates gammadelta T cells from murine genital tract", Vaccine, vol. 28, no. 29, May 2010 (2010-05), pages 4600-4608,
- HEIDEMAN, DANIELLE AM et al.: "Oncolytic adenovirus expressing a p53 variant resistant to degradation by HPV E6 protein exhibits potent and selective replication in cervical cancer", Molecular Therapy, vol. 12, no. 6, 2005, pages 1083-1090,
- BALAGUÉ, CRISTINA et al.: "Human papillomavirus E6E7-mediated adenovirus cell killing: selectivity of mutant adenovirus replication in organotypic cultures of human keratinocytes", Journal of virology, vol. 75, no. 16, 2001, pages 7602-7611,
- GAMBOTTO, ANDREA et al.: "Induction of antitumor immunity by direct intratumoral injection of a recombinant adenovirus vector expressing interleukin-12", Cancer gene therapy, vol. 6, no. 1, 1999, pages 45-53,
- CROYLE, MARIA A. et al.: "Nasal delivery of an adenovirus-based vaccine bypasses pre-existing immunity to the vaccine carrier and improves the immune response in mice", PloS one, vol. 3, no. 10, 2008, page E3548,
- RUDIN, CHARLES M. et al.: "An attenuated adenovirus, ONYX-015, as mouthwash therapy for premalignant oral dysplasia", Journal of clinical oncology, vol. 21, no. 24, 2003, pages 4546-4552,
- APPLEDORN, DANIEL M. et al.: "Sublingual administration of an adenovirus serotype 5 (Ad5)- based vaccine confirms Toll-like receptor agonist activity in the oral cavity and elicits improved mucosal and systemic cell -mediated responses against HIV antigens despite preexisting Ad5 immunity", Clinical and Vaccine Immunology, vol. 18, no. 1, January 2011 (2011-01), pages 150-160,
- BABIUK, L. A. et al.: "Adenoviruses as vectors for delivering vaccines to mucosal surfaces", Journal of biotechnology, vol. 83, no. 1, 2000, pages 105-113,
- DIAZ-ARRASTIA , CONCEPCION et al.: "Clinical and molecular responses in high-grade intraepithelial neoplasia treated with topical imiquimod 5%", Clinical cancer research, vol. 7, no. 10, 2001, pages 3031-3033,
- MEYSKENS, FRANK L. et al.: "Prevention of cervical intraepithelial neoplasia and cervical cancer", The American journal of clinical nutrition, vol. 62, no. 6, 1995, pages 1417S-1419S,
- "the cervix", Canadian Cancer Society, Retrieved from the Internet: URL:http://www.cancer.ca/en/cancer-informa tion/cancer-type/cervical/cervical-cancer/ the-cervix/?region=on
- "NCI Dictionary of Cancer terms", NIH, page 1,
- DOORBAR J: "The papillomavirus life cycle", Journal of Clinical Virology, vol. 32S, 2005, pages S7-S15,
- DOORBAR J et al.: "The Biology and Life-Cycle of Human Papillomaviruses", Vaccine, 2012, pages F55-F70,
- Zervixzytology (Auf dem Laufenden, 2016), (https://twitter.com/eurocytology)
- Schiffman M.; Wentzensen N.: "Human Papillomavirus (HPV) infection and the multi-stage carcinogenesis of cervical cancer", Cancer Epidemiol Biomarkers Prev., vol. 22, no. 4, 2013, pages 553-560,
- Cuburu N. et al: "Adenovirus vector-based prime-boost vaccination via heterologous routes induses cervicovaginal CD8+ T cell responses against HPV16 oncoproteins", International Journal of Cancer, 2017, pages 1-13,

## Beschreibung

Die Erfindung betrifft ein Mittel zur lokalen Behandlung von Zervixdysplasien, umfassend einen rekombinanten, genetisch modifizierten in nicht-HPV-infizierten Zellen replikationsdefekten Adenovirus.

### Stand der Technik und Hintergrund der Erfindung

Hintergrund der Erfindung sind Erkrankungen, die von humanen Papilloma-Viren (HPV) hervorgerufen werden, insbesondere Gebärmutterhalskrebs und seine Vorstufen.

Allein in Deutschland werden pro Jahr ca. 1 Mio pathologische Befunde an Frauen hinsichtlich HPV-induzierter neoplastischen Veränderungen des Gebärmutterhalses (*cervix uteri*) erhoben. Bei einem großen Teil der betroffenen Frauen heilt die Läsion ohne weitere Maßnahmen im Verlauf einiger Wochen bis Monate ab. Tritt jedoch keine Spontanheilung ein und kommt es zur Entwicklung hochgradig dysplastischer Epithelveränderungen, wird von ärztlicher Seite aufgrund des erhöhten Risikos einer Progression zum Zervixkarzinom die chirurgische Entfernung des betroffenen Areals empfohlen (Konisation). Gegenwärtig existiert für Frauen mit höhergradigen zervikalen intraepithelialen Neoplasien (CIN) keine therapeutische Alternative zur chirurgischen Abtragung des dysplatischen Epithels (Konisation). Jährlich müssen sich ca. 150.000 Frauen in Deutschland einer Konisation unterziehen. Für die betroffenen Frauen sind diese Eingriffe nicht nur belastend, sondern erhöhen bei späteren Schwangerschaften auch das Risiko für eine Frühgeburt. Die auch in Deutschland kürzlich eingeführte prophylaktische Impfung mit den neuentwickelten Impfstoffen Gardasil® (Merck) und Cervarix® (GlaxoSmit-hKline) verhütet zuverlässig bei jungen Menschen, die noch keinen Geschlechtsverkehr hatten, eine Erstinfektion mit HPV 6, 11, 16 und 18 (bei Gardasil®). Ist ein Mensch jedoch bereits mit HPV-Viren infiziert, hat der Impfstoff keine Wirkung mehr. Entsprechend liegt seine prophylaktische Wirksamkeit zur Verhütung von zervikalen intraepithelialen Neoplasien (CIN-Läsionen) bei 25jährigen Frauen unabhängig vom verursachenden HPV-Serotyp nur noch bei 17%. Da der erste Erkrankungsgipfel beim Zervixkarzinom zwischen dem 35. und dem 40. Lebensjahr liegt, wird es selbst bei breiter Anwendung einer prophylaktischen Impfung noch ca. 20 Jahre dauern, bis die Wirkung der Impfung merklichen Einfluss auf die Zervikarzinominzidenz nimmt. Die Notwendigkeit regelmäßiger Vorsorgeuntersuchungen und der Wunsch nach wirksamen, schonenderen Therapiemaßnahmen wird trotz der Einführung der prophylaktischen Impfung bestehen bleiben.

Ursache für die Entstehung von Zervixdysplasien sind persistierende Infektion sind Hochrisiko (high risk) Papillomaviren. Bei jungen Frauen in der Altersgruppe zwischen 20 und 30 Jahren erreicht die Inzidenz von Hochrisiko-HPV-Infektionen bis zu 50% (so in Dänemark). In der Folge entwickeln infizierte Frauen regelmäßig zytologische Auffälligkeiten beim sog. Zervixabstrich (Pap-Abstrich) bzw. dysplastische Veränderungen im Sinne einer zervikalen intraepithelialen Neoplasie (CIN) bis hin zu Zervixkarzinomen. Obwohl die Ursache der dysplastischen Veränderungen eine Virusinfektion ist, erfolgt die immunologische Abwehrreaktion häufig verspätet oder ist in ihrer Wirkung unzureichend, so dass es zu protrahierten Infektionen kommt, die sich über Zeiträume von Monaten bis zu mehreren Jahren erstrecken können. Ursache für die unzureichende immunologische Abwehrreaktion sind niedrige Replikationsraten und in der Folge nur geringe Mengen an viralen Antigenen, die in den Zielorganismus gelangen und zusätzlich effektive HPV-Mechanismen zur Unterdrückung von immunologischen Abwehrreaktionen. Da Papillomaviren insbesondere Zellen der sog. Transformationszone im Bereich der Zervix uteri infizieren, sind die induzierten Gewebeveränderungen meist auf den Bereich des distalen Zervixkanals und zentrale Anteile der Portio uteri beschränkt.

Im Stand der Technik sind weiterhin Methoden zur Zerstörung von HPV-Onkoprotein-exprimierender Zellen zu finden, die zur Prophylaxe und/oder zur Behandlung von HPV-induzierten Erkrankungen, wie etwa Gebärmutterhalskrebs und seinen Vorstufen, geeignet sind.

So beschreibt die WO 2009/106362 A1 ein Auswahlverfahren für Nukleinsäuren, die für ein oder mehrere Humanpapilloma-Virus (HPV)-Onkoproteine oder Fragmente davon kodieren. Dabei stellt sich die WO 2009/106362 A1 die Aufgabe eine vermeintlich sichere Vakzine dadurch zu schaffen, dass diese nur antigene Sequenzmotive aufweist, dass heißt, dass alle anderen Sequenzmotive wie solche mit transformations-assoziierten Peptidmotiven aus der DNA der Vakzine durch Klonierungsmassnahmen zuvor entfernt wurden. Eine solche Nukleinsäure soll nach Expression in einem Säugerorganismus eine Immunantwort induzieren können, welche zur Zerstörung von HPV-Onkoprotein-exprimierender Zellen führt und damit zur Prophylaxe und/oder Behandlung von HPV-induzierten Erkrankungen, wie etwa Gebärmutterhalskrebs und seinen Vorstufen, geeignet ist.

Hoffmann et al. (J. Immunother 2010; 33: 136-145) beschreiben einen ähnlichen Ansatz zur Behandlung von HPV-induzierten Erkrankungen mit einer rekombinanten DNA-Vakzine, nämlich einer Adenovirus-basierenden T-Zell Vakzine, bei der problematische Sequenzmotive deletiert wurden, die eine spezifische T-Zell-Antwort in vivo hervorrufen sollen.

Diesen Methoden ist gemeinsam, dass es sich um Vakzinierungsmittel handelt, also um Impfstoffe, die mithin direkt in die Blutbahn gelangen.

### Ziel der Erfindung

Ausgehend von diesem Stand der Technik ist es die Aufgabe der vorliegenden Erfindung, eine alternative Therapiemöglichkeit zur Behandlung von HPV-induzierten Gewebeveränderungen (Zervixdysplasien) bereitzustellen.

### Beschreibung und Vorteile der Erfindung

Gelöst wird diese Aufgabe durch die Merkmale der selbstständigen Ansprüche; vorteilhafte weitere erfindungsgemäße Maßnahmen sind in den übrigen Unteransprüchen enthalten.

Die Erfindung wird nunmehr im Einzelnen wie folgt dargestellt:
Die Erfindung betrifft ein Mittel zur Verwendung als Medikament zur Behandlung von Zervixdysplasien, umfassend einen rekombinanten, genetisch modifizierten in nicht-HPV-infizierten Zellen replikationsdefekten E1-deletierten Adenovirus, wobei das Mittel lokal und äußerlich im Bereich der Portio und Zervix uteri verabreicht wird und der E1-deletierte Adenovirus ein oder mehrere Papillomavirus-Antigenepitope repräsentierende Vakzinierungsgene umfasst, deren Expression geeignet ist eine zelluläre oder humorale Immunantwort gegen HPV-infizierte oder HPV-Onkogen exprimierende Zellen zu erzeugen.

Der Erfindung liegt die überraschende Erkenntnis zugrunde, dass die Nutzung der spezifischen Wechselwirkung zwischen HPV-Onkogenen und E1-deletierten Adenoviren zu einem replizierenden therapeutisch nutzbaren System, in einzigartiger Weise zur therapeutischen Behandlung von Zervixdysplasien geeignet ist. Da im Gebärmutterhals eine direkte Zugänglichkeit dysplastischer Zellen gegeben ist, besteht die Möglichkeit Onkogen-exprimierende dysplastische Zellen direkt zu erreichen und durch selektive Replikation zu zerstören. Im Unterschied zu einer Injektion ist hier also die äußerliche, lokale Applikation besonders bevorzugt. Gleichzeitig führt die Replikation des Adenovirusgenoms in HPV-Onkogen-exprimierenden Zellen zu einer verstärkten Expression des Vakzinierungsgens und lenkt zusätzlich durch die vektoreigene (adenovirale) Antigenität die lokale und systemische Immunreaktivität auf die dysplastischen Zellen, es wird also gleichzeitig eine zusätzliche Verstärkung der sytemischen, onkogen-spezifischen Immunantwort induziert.

Besonderes Merkmal der Erfindung ist daher die lokale Anwendung rekombinanter Adenoviren im Kontext einer persistierenden HPV-Infektion im Bereich der Portio und Zervix uteri. Dem liegt die überraschende Erkenntnis zugrunde, dass nur die lokale Anwendung rekombinanter Adenoviren, in Abgrenzung zu einer systemischen Vakzinierung, es erlaubt die biologischen Besonderheiten im Wirkungsgeflecht zwischen E1-deletierten Adenoviren und HPV-infizierten bzw. HPV-Onkogen exprimierenden Körperzellen therapeutisch zu nutzen.

Das therapeutische Prinzip der vorliegenden Erfindung beruht auf einer künstlichen Infektion der dysplastischen Zellen des Gebärmutterhalses mit einem ungefährlichen, aber stark immunogenen Virus. Das therapeutische Virus trägt in seinem Genom HPV-spezifische Impfgene und lenkt durch seine eigene Immunogenität und durch die Expression der Impfgene die Aufmerksamkeit des Immunsystems auf alle HPV-infizierten Zellen und hilft, die HPV-Infektion zur Abheilung zu bringen. Zu diesen beiden getrennt bereits gut untersuchten therapeutischen Mechanismen (Yang et al., Proc Natl Acad Sci U S A. 1994 May 10; 91 (10): 4407-1, Hoffmann et al., J Immunother. 2010 Feb-Mar; 33 (2): 136-45) tritt ein drittes synergistisches Wirkprinzip hinzu, das die Anwendung eines E1-deletierten replikationsdefekten Adenovirus zur lokalen Behandlung von HPV-Onkogen-exprimierenden Zervixdysplasien zu einem einzigartigen Wirkprinzip macht: In einer Körperzelle, in der die beiden HPV-Onkogene E6 und E7 exprimiert werden, führt die Infektion mit einem E1-deletierten Adenovirus zu einer selektiven Replikation der das Vakzinierungsgen tragenden adenoviralen DNA. Dieser synergistische Mechanismus ist zwar prinzipiell bekannt und für die intratumorale Applikation und Behandlung von Zervixkarzinomen vorgeschlagen worden (Steinwaerder et al., Mol Ther. 2001 Sep; 4(3): 211-6), allerdings ist bislang nicht erkannt worden, dass dieses Wirkprinzip sein Potential aufgrund der im Unterschied zu soliden Tumoren anderen anatomischen und infektiologischen Situation von Zervixdysplasien, hier nachhaltiger entfalten kann. Die vorliegende Erfindung macht sich daher das von Steinwaerder et al. (Mol Ther. 2001 Sep; 4(3): 211-6) beschriebene biologische Phänomen zur Behandlung persistierender HPV-Infektionen und früher prämaligner dysplastischer Epithelläsionen im Bereich der Cervix uteri zunutze. Denn im Unterschied zu dem kompakten Gewebe von Zervixkarzinomen, in denen alle Zellen HPV E6 und E7 Onkogene hoch exprimieren, umfassen die Epithelien der Cervix uteri meist nur wenige Zellschichten, in denen nur einzelne disseminiert stehende Zellen HPV Onkogene exprimieren. Denn insbesondere handelt es sich bei Zervixdysplasien um eine flächige Hautveränderung, die in ihrer horizontalen Ausdehnung nur wenige Mikrometer umfasst, und auf die äußerste Hautschicht, das Epithel, beschränkt ist.

Der Synergismus zwischen E1-deletiertem Adenovirus und HPV-infizierter Epithelzelle, den sich die Erfindung zunutze macht, kann wie folgt beschrieben werden:
Die Erfindung geht von der - durchaus bekannten - Erkenntnis aus, dass rekombinante replikationsdefekte Adenoviren das leistungsfähigste *in vivo* Gentransfersystem der vergangenen 20 Jahre darstellen. Adenoviren sind bislang bereits in mehr als 150 klinischen Studien als Vektorsystem zum Transfer therapeutischer Gene bzw. zur Expression von Vakzinierungsgenen eingesetzt worden. Wildtyp-Adenoviren sind unbehüllte DNA-Viren die eine in etwa 36.000 Basenpaare lange doppelsträngige DNA tragen und typischerweise leichte Infektionen der oberen Atemwege erzeugen. Die zu therapeutischen Zwecken in der Medizin genutzten replikationsdefekten E1-deletierten rekombinanten Adenoviren sind durch eine etwa 3.000 Basenpaare umspannende Deletion der adenoviralen E1-Region charakterisiert. Diese Viren sind in der Lage fast jede humane Körperzelle sehr effektiv zu infizieren, können sich aber in dieser Zelle durch das Fehlen der E1-Gene (E1A und E1B-Gene) nicht selber replizieren. Die Herstellung bzw. Produktion dieser Viren erfordert die Nutzung spezieller Zelllinien, die die adenoviralen E1-Gene in ihrem Genom tragen und auf diese Weise ihre Funktion indirekt (*in trans*) bereitstellen. Durch die Deletion der E1-Gene im adenoviralen Genom entsteht eine "Lücke", in die Fremdgene (therapeutische Gene oder Vakzinierungsgene) einkloniert werden können und die nach Infektion einer Körperzelle dort exprimiert werden. Um zusätzlichen Raum für die Klonierung größerer Transgene zu schaffen, tragen erweiterte Varianten rekombinanter Adenoviren eine zweite Deletion im Bereich der adenoviralen E3-Region, die das Einklonieren von Transgenen bis zu einer Gesamtgröße von etwa 7.800 Basenpaaren erlaubt. Die Plasmide, die für die Herstellung der im Rahmen dieser Erfindung verwendeten Viren eingesetzt werden, sind im Labor von Frank Graham (McMaster University, Hamilton, Ontario, Canada) in den 80iger Jahren des letzten Jahrhunderts entwickelt worden (McGrory WJ et al., Virology 1988, 163 (2): 614-7).

Wenn auch die funktionelle Präsens der adenoviralen E1-Gene für eine Hochreplikation und Entstehung neuer infektiöser Adenoviren essentiell ist, kann die Funktion der adenoviralen E1-Gene von anderen Onkogenen bis zu einem gewissen Maß komplementiert werden, was zu einer begrenzten genomischen Replikation des adenoviralen Genoms in onkogen-exprimierenden Zellen führt. Unter diesen zur Transaktivierung fähigen Onkogenen nehmen die papillomviralen Onkogene E6 und E7 eine besondere Stellung ein, da sie eine nachhaltige Transaktivierung herbeiführen können, die zu einer Replikation des adenoviralen Genoms bis zu mehreren hundert Kopien pro Zelle führt. Auf diese Weise erfolgt auch die Expression des Transgens (Vakzinierunsgen, therapeutsches Gen) nicht nur aus einer oder wenigen Kopien, sondern aus mehreren Hundert Kopien gleichzeitig, was die Gesamtstärke der Transgenexpression dramatisch vestärkt.

Dies wiederum macht sich die Erfindung zunutze; denn ein in das Adenovirusgenom integriertes "therapeutisches" Gen oder Vakzinierungsgen (wie im Rahmen dieser Erfindung) wird dann nicht nur aus einigen wenigen Kopien, sondern aus mehreren Hundert Kopien exprimiert. Dieser Mechanismus wiederum führt zu einer erheblichen Steigerung der Expressionshöhe des therapeutischen Gens bzw. Vakzinierungsgens und induziert gleichzeitig den Untergang der HPV-infizierten Zelle durch die Überexpression adenoviraler Antigene.

Innerhalb des Kollektivs HPV-infizierter Zellen exprimiert in der Regel nur ein Teil der Zellen die HPV-Onkogene E6 und E7. Von diesen Zellen geht allerdings die größte Gefahr für die Entstehung schwerer Dysplasien und später der Entstehung von Gebärmutterhalskrebs aus. Bei einer erfindungsgemäßen flächendeckenden, direkten lokalen äußerlichen Infektion HPV-infizierter Zellen des Gebärmutterhalses mit einem E1-deletierten Adenovirus werden jedoch gerade HPV-Onkogen exprimierende Zellen das Vakzinierungsgen aufgrund des oben beschriebenen Mechanismus besonders stark exprimieren. Die verstärkte Replikation des adenoviralen Genoms in HPV-Onkogen exprimierenden Zellen trägt damit auf zweierlei Weise zum therapeutischen Effekt bei. Zum einen führt die Replikation zu einer Verstärkung der T-Zellantwort durch verstärkte Expression des Vakzinierungsgens und damit der Bereitstellung von Zielantigen und zum zweiten führt die kontinuierliche Replikation des adenoviralen Genoms zum Niedergang der HPV-Onkogen exprimierenden Zelle. Auf diese Weise werden gerade die Zellen, von denen die größte Gefahr für die Entstehung dysplastischer Zellveränderungen ausgeht, selektiv zerstört. Der Erfindung liegt daher die Erkenntnis zugrunde, dass weder eine systemische Applikation noch eine lokale Injektion dazu geeignet ist, eine flächendeckende Infektion HPV-infizierter Gewebe zu gewährleisten. Im Gegenteil sollte sogar jede lokale Blutung - wie sie durch eine Injektion geschieht - vermieden werden, um einen Kontakt der "therapeutischen" Viren gemäß der Erfindung mit neutralisierenden Antikörpern zu verhindern. Gemäß der Erfindung erlaubt es daher nur eine äußerliche Anwendung die dysplastischen Läsionen einschließlich ihres Ursprungsgewebes im Bereich der Transformationszone und des distalen Zylinderepithels vollständig zu infizieren.

In der späten Infektionsphase HPV-infizierter Körperzellen kommt es zu einer verstärkten Expression der beiden HPV-Onkogene E6 und E7. Die Expression dieser beiden Onkogene führt zu einer Beschleunigung des Zellzyklus bei gleichzeitiger Hemmung zellulärer Tumorsuppressorgene (insbesondere p53 und pRb). Die Expression dieser beiden HPV-Onkogene ist ursächlich für die Entwicklung dysplastischer Epitheldefekte der Zervix uteri und letztendlich für die Entwicklung von Gebärmutterhalskrebs verantwortlich. Die Aufhebung der Expressionshemmung der HPV-Onkogene stellt daher einen entscheidenden Pathomechanismus der Dysplasieentstehung dar.

Entscheidend für die Pathogenese von Zervixdysplasien ist die Expression der beiden HPV-Onkogene E6 und E7. Diese Onkogene stimulieren den Zellzyklus der infizierten Zelle auf vielfältige und unphysiologische Weise. Wird in dieser Situation eine HPV-Onkogen exprimiernde Zelle allerdings mit einem E1-deletierten Adenovirus infiziert, führt die beginnende Replikation des adenoviralen Genoms und die Überexpression der Transgene zu einer langsamen Überladung der infizierten Zelle mit adenoviralen- bzw. transgenen Expressionsprodukten und treibt die dysplastische Zelle selektiv in den Zelltod. Gleichzeitig induziert der Replikationsmechansimus auch eine Steigerung der Expressionshöhe des Vakzinierungsgens und der induzierten Immunantwort.

Da das im Rahmen dieser Erfindung verwendete Adenovirus ein Vakzinierungsgen trägt, dessen Expression in der Lage ist eine spezifische Immunantwort gegen HPV-Onkogene zu induzieren, führt die Infektion einer E6/E7 exprimierenden Zelle über den oben beschriebenen Replikationsmechanismus zu einer zusätzlichen Verstärkung der Vakzinierunsgenexpression und damit zu einer weiteren Steigerung der zellulären Immunität gegen HPV-Onkogen exprimierende Zellen.

Grundsätzlich ist aber die beschriebene funktionelle Synergie zwischen HPV- E6/E7 Onkogenen und E1-deletierten Adenoviren von der Natur des Transgens unabhängig, so dass dieses Prinzip auch zur Überexpression anderer therapeutischer Transgene genutzt werden könnte.

Die Erfindung eröffnet damit einen völlig neuartigen Therapieansatz zur Behandlung von Zervixdysplasien. Ein lokaler äußerlicher Auftrag des erfindungsgemäßen Mittels, enthaltend rekombinante E1-deletierte Adenoviren im Bereich der Portio und Zervix uteri führt zu einer erfolgreichen Behandlung von HPV-induzierten lokalen Gewebeveränderungen.

Die Erfindung kommt also ohne einen chirurgischen Eingriff am Patienten aus, was selbstredend ein gravierender Vorteil gegenüber vergleichbaren Methoden darstellt, da es die körperliche Unversehrtheit des Patienten gewährleistet.

Erfindungsgemäß ist bevorzugt, dass rekombinante Adenoviren Verwendung finden, die für ein oder mehrere Vakzinierungsgene bzw. immunmodulatorische Gene kodieren, deren Expression wiederum geeignet ist eine zelluläre oder humorale Immunantwort gegen HPV-infizierte oder HPV-Onkogen exprimierende Zellen zu erzeugen bzw. die geeignet sind eine verstärkte Immunantwort gegen HPV-infizierte Zellen zu erzeugen. Bevorzugt sind Vakzinierungsgene, die eine Immunantwort gegen die Human Papilloma Viren (HPV) 16, 18, 25, 31, 33, 35, 39, 45, 51, 52, 53, 56, 58, 59, 66, 68, 73 oder 82 hervorrufen. Dabei sind besonders bevorzugt solche Vakzinierungsgene, die eine Immunantwort gegen die Human Papilloma Viren HPV16 und HPV18 hervorrufen.

Mit der aktiven Replikation therapeutischer Adenoviren in HPV-Onkogen exprimierenden Zellen ist zum einen eine verstärkte Expression der codierenden Transgene und damit eine verstärkte immunologische Wirkung verbunden und zum anderen führt die aktive Replikation des therapeutischen Virus in HPV E6/E7 exprimierenden Zellen zu einer verstärkten lokalen Freisetzung therapeutischer Viren in Bereiche, an den sie aus therapeutischer Sicht am dringlichsten notwendig ist.

Der durch die Erfindung bereitgestellte vorteilhafte therapeutische Effekt bei einer äußeren Anwendung im Bereich der Portio und der Cervix uteri aufgebrachter E1-deletierter rekombinanter Adenoviren setzt sich aus mehreren Komponenten zusammen:
Die Infektion einer Säugerzelle mit einem E1-deletierten Adenovirus führt regelmäßig zu einer spezifischen Aktivierung des zellulären Immunsystems, die eine Inaktivierung oder Eliminierung der Adenovirus-infizierten Zelle nach sich zieht. Eine lokale Infektion des dysplastischen Epithels mit rekombinanten Adenoviren wird durch diesen Mechanismus bereits zu einer teilweisen Eliminierung dysplastischer Zellen führen. Da die Epithelien im Bereich dysplastischer Läsionen regelmäßig mit Hochrisiko Papillomaviren infiziert sind, ist mit einer Adenovirus-vermittelten Zerstörung dieser Zellen auch eine verstärkte Freisetzung von HPV-Antigenen verbunden. Die Zerstörung und Phagozytose HPV-infizierter Zellen führt regelmäßig auch zu einer verstärkten Präsentation papillomaviraler Antigene und damit zu einer verstärkten Immunreaktion gegen HPV-infizierte Zellen.

Anders als eine HPV-infizierte Zelle führt die Infektion einer (nicht infizierten) Zelle mit einem replikationsdefizienten Adenovirus also immer zuverlässig zu einer Reaktion des zellulären Immunsystems, in dessen Folge die infizierte Zelle inaktiviert bzw. zerstört wird. Diese in der Regel unerwünschte Reaktion macht die Nutzung rekombinanter Adenoviren als Transportvehikel (Vektor) für therapeutische Gene zur dauerhaften Korrektur monogenetischer Erkrankungen nahezu unmöglich.

Bei der erfindungsgemäßen Behandlung von Zervixdysplasien jedoch ist die hohe Antigenität gerade erwünscht, da sie die mangelnde Antigenität HPV-infizierter Zellen kompensieren kann und eine Abwehrreaktion des Immunsystems erzwingt. Die Erfindung macht sich dies zunutze, indem sie diesen Mechanismus im Rahmen einer lokalen direkten äußerlichen Infektion der Zellen zur Entfaltung bringt. Eine derartige Verwendung rekombinanter Adenoviren zur Lokalbehandlung von dysplastischen Veränderungen der Zervix uteri hat bislang nicht stattgefunden.

Da bei der Mehrzahl junger Erwachsener Antikörper gegen Serotyp 5 Adenoviren nachgewiesen werden können, stellt die erfindungsgemäße lokale äußerliche Adenovirusinfektion im Bereich der Portio und Zervix uteri einen Applikationsweg dar, der von den infektionshemmenden Einflüssen neutralisierender Antikörper weitgehend unabhängig ist, da durch die gute Zugänglichkeit der Epithelien sezernierte Antikörper vor einer therapeutischen Infektion leichter entfernt werden können. Nur eine äußerliche Anwendung der im Rahmen der Erfindung eingesetzten Adenoviren erlaubt es die dysplastischen Läsionen einschließlich ihres Ursprungsgewebes im Bereich der Transformationszone und des distalen Zylinderepithels umfassend zu infizieren.

Die Erfindung beruht daher darauf, das HPV-infizierte Gewebe äußerlich mit dem erfindungsgemäßen Mittel zu behandeln, was zu einer Infektion des HPV-infizierten Gewebes mit rekombinanten E1-deletierten Adenoviren führt, die sich in HPV E6/E7 exprimierenden Zellen (wie dem infizierten Gewebe) replizieren können. Diese jedoch können sich nur und ausschließlich in diesem infizierten Gewebe vermehren, da derartige Zellen mangelnde Antigenität aufweisen und somit eine gezielte Immunantwort hervorrufen.

In einer besonderen Ausführungsform der Erfindung ist vorgesehen im Rahmen einer lokalen Therapie von Zervixdysplasien rekombinante E1-deletierte Adenoviren einzusetzen, die mindestens ein oder zwei Vakzinierungsgene tragen. Diese Vakzinierunsgene repräsentieren wichtige Papillomavirus-Antigenepitope. Nach Infektion einer Zelle mit einem rekombinanten Adenovirus werden diese Gene stark exprimiert und es entstehen in kurzer Zeit ein Papillomavirus-Antigenpool, der die im Rahmen einer HPV-Wildtypinfektion entstehende Antigenmenge überschreiten kann. Auf diese Weise erzwingt die Expression dieser Gene im Kontext einer Adenovirusinfektion eine spezifische zelluläre Immunantwort sowohl gegen Papillomavirusonkogene und Papillomavirus-Hüllproteine. Die Wirksamkeit dieser Maßnahme konnte an HPV-Onkogen exprimierenden Maustumormodellen gezeigt werden.

Zur Anwendung gelangen hier Vakzinierungsgene, insbesondere das Vakzinierungsgen "p14", deren prinzipielle Herstellung in der WO 2009/106362 A1 (dort Beispiel 1: "Klonierung eines rekombinanten therapeutischen Vakzinierungsgens ohne Transformations-assoziierte Peptidmotive") sowie in Hoffmann et al. (J. Immunother 2010; 33: 136-145) beschrieben ist. Hierauf, sowie auf den gesamten Offenbarungsgehalt der WO 2009/106362 A1 und Hoffmann et al. (J. Immunother 2010; 33: 136-145) wird Bezug genommen und als Referenz in die vorliegende Anmeldung inkorporiert.

Das dort beschriebene rekombinante Vakzinierungsgen (p14) zeichnet sich dadurch aus, dass alle potentiell pathogenen und transformierenden Sequenzabschnitte eliminiert wurden. Auch hat eine Analyse der im rekombinanten Vakzinierungsgen p14 noch codierten T-Zellepitope (bezogen auf die 4 in der kaukasischen Bevölkerung häufigsten MHC-I Moleküle) gezeigt, dass trotz der Eliminierung aller potentiell pathogenen und transformierenden Sequenzabschnitte, nahezu 70% der hochaffinen T-Zellepitope vom Vakzinierunsgen noch codiert werden. Ferner konnte in Hinblick auf das rekombinate Vakzinierungsgen (p14) mittels Transformationsassays zum Ausschluss einer noch im Vakzinierunsgen verbliebenden transformierenden Restaktivität gezeigt werden, dass die stabile Expression des Vakzinierungsgens p14 (pCMV-p14) in NIH-3T3 Zellen im Unterschied zu den Wildtyponkogenen keinerlei transformierende Restaktivität mehr zeigt.

Zum Nachweis der Fähigkeit des Vakzinierunsgens nach Expression in Säugern eine spezifische gegen HPV-Onkogene gerichtete Immunantwort aufzubauen, wurde Mäusen (C57BL6) durch subkutane Injektion 1x10⁶ C3-Tumorzellen transplantiert. C3-Tumorzellen exprimieren die HPV-Onkogene E6 und E7. 7 Tage nach Tumortransplantation wurde den Mäusen einmalig eine Dosis von 1 x 10¹⁰ infektiöse Partikel (i.p) in 200 µl Injektionspuffer intramuskulär verabreicht. In allen 10 in dieser Weise behandelten Mäusen kam es zu einer vollständigen und dauerhaften Abheilung der Tumore, während die Tumore in der Kontrollgruppe (nach Applikation eines Kontrollvirus (Ad-lacZ) in 9 von 10 Fällen weiter auswuchsen (vgl. WO 2009/106362 A1 und Hoffmann et al., J. Immunother 2010; 33: 136-145). Es konnte also gezeigt werden, dass die Expression des rekombinanten Vakzinierungsgens nach einmaligem adenoviralem Gentransfer (intramuskuläre Applikation 7 Tage nach Tumortransplantation) in Säugern (C57BL6-Mäuse) eine T-Zellantwort auslöst, die ausreicht bereits angewachsene C3-Tumoren vollständig und dauerhaft zu zerstören. In allen 10 tumortragenden Versuchstieren führte die einmalige Vakzinierung mit Ad-p14 zur dauerhaften Zerstörung der Tumore, während in 9 von 10 Versuchtieren in der Kontrollgruppe (Applikation von Ad-lacZ) die Tumoren nach Virusapplikation ungehindert weiterwuchsen.

Dieses Vakzinierungsgen hat sich in Bezug auf die vorliegende Erfindung überraschend als besonders wirkungsvoll erwiesen.

Die vorliegende Erfindung macht sich daher diese Erkenntnis zunutze, dass E1-deletierte Adenoviren sich in HPV E6/E7 exprimierenden Zellen replizieren können und somit gezielt die Zerstörung HPV-infizierter Epithelien unterstützen. Denn auf diese Weise kommt es zur einer verstärkten und gezielten Zerstörung von HPV-infizierten Zellen, die ein erhöhtes Risiko für eine Dysplasieentwicklung tragen und gleichzeitig ist mit der Replikation eine verstärkte Expression der Vakzinierungsgene und damit eine verstärkte Immunantwort verbunden.

Das bevorzugt zur Anwendung kommende Vakzinierungegen ist ein solches, welches durch Fusion von Bruchstücken der HPV-Onkogene E6 und E7 der *high risk* HPV 16 und HPV 18 hergestellt wurde (Kurzname: "p14"). Insofern ist auch die korrespondierende DNA-Sequenz (1248 bp, SEQ ID No. 1) des Vakzinierungsgens p14 Gegenstand der Erfindung. Dieses Vakzinierungsgen p14 kodiert für ein Protein mit der Aminosäuresequenz gem. SEQ ID No. 2 (415 Aminosäuren).

Erfindungsgemäß ist weiterhin bevorzugt, dass der E1-deletierte Adenovirus ein oder mehrere immunmodulatorische Gene umfasst, die geeignet sind eine verstärkte Immunantwort gegen HPV-infizierte Zellen zu erzeugen. Hierbei sind Interleukine zur Immunmodulation bevorzugt, wobei die Erfindung nicht beschränkt ist auf Interleukine, sondern alle üblichen gleichwirkenden Immunmodulatoren ebenfalls umfasst. Besonders bevorzugt sind inflammatorische Interleukine, wie beispielsweise IL1, IL-2 oder IL-12.

Der Vorteil der Erfindung liegt darin, dass die lokale Zerstörung HPV-infizierter Zellen durch die Lokalbehandlung mit rekombinanten Adenoviren zu einer verstärkten Freisetzung papillomaviraler Antigene führt und somit die Abwehrreaktion gegen virale Antigene gefördert wird.

Die optimale Entfaltung und das optimale Zusammenspiel der beschriebenen Mechanismen zur Behandlung von Zervixdysplasien setzt die Anwendung eines Verfahrens voraus, dass den Einfluss blutständiger neutralisierender Antikörper gegen Adenoviren minimiert. Dies ist nur durch den lokalen äußerlichen Auftrag rekombinanter E1-deletierter Adenoviren im Bereich der Portio und Zervix uteri möglich, da hier aufgrund der guten Zugänglichkeit eine der lokalen Infektion vorausgehende Konditionierung des lokalen Epithels möglich ist und auf diese Weise sekretierte Antikörper entfernt und optimale lokale Infektionsraten gewährleistet sind.

Gegenstand der Erfindung ist auch eine pharmazeutische Zusammensetzung bzw. ein Arzneimittel zur Anwendung am Menschen zur lokalen Behandlung von Zervixdysplasien, umfassend einen rekombinanten, genetisch modifizierten in nicht HPV-infizierten Zellen replikationsdefekten Adenovirus.

Die Erfindung stellt mithin ein therapeutisch wirksames Mittel, insbesondere eine pharmazeutisch wirksame Zusammensetzung zur Verfügung, die als Arzneimittel zur lokalen äußeren Anwendung von HPV-induzierten lokalen Gewebeveränderungen, insbesondere zur lokalen äußerlichen Anwendung im Bereich der Portio und Zervix uteri, geeignet ist, umfassend rekombinante replikationsdefekte E1-deletierte Adenoviren. In bevorzugter Ausführungsform umfassen diese auch ein oder mehrere Papillomavirus-Antigenepitope repräsentierende Vakzinierungsgene, deren Expression geeignet ist eine zelluläre oder humorale Immunantwort gegen HPV-infizierte oder HPV-Onkogen exprimierende Zellen zu erzeugen. Eine dementsprechende pharmazeutische Zusammensetzung umfassend das erfindungsgemäße Mittel weist die notwendigen galenischen Zusatzstoffe auf, die zur Herrichtung und Verabreichung des Arzneimittels zur äußerlichen lokalen Anwendung im Bereich der Portio und Zervix uteri geeignet sind.

Das erfindungsgemäße Mittel kann alleine oder kombiniert mit mindestens einer weiteren pharmazeutisch wirksamen Substanz, beispielsweise einer weiteren pharmazeutisch wirksamen Nukleinsäure, beispielsweise ein inflammatorisches Interleukin und/oder auch ein costimulatorisches Molekül, verabreicht werden. Beispiele für costimulatorische Moleküle sind in und in US 2006/0171949 beschrieben, auf deren Offenbarung hiermit ausdrücklich Bezug genommen wird. In einer besonders bevorzugten Ausführungsform ist das Molekül ein Aktivator des GITR-Signalwegs, insbesondere ein an den GITR-Rezeptor bindender agonistischer Antikörper oder eine lösliche Form des GITR-Liganden (GITR-L). Beispiele für entsprechende GITR-Bindemoleküle sind in US 2007/0098719 beschrieben, auf deren Offenbarung hiermit ausdrücklich Bezug genommen wird. Weiterhin kann die erfindungsgemäße Vakzine auch zusammen mit Adjuvantien, wie etwa Bakterientoxinen, z.B. Choleratoxin oder hitzestabiles E. coli Enterotoxin, chemischen Adjuvantien oder mit Cytokinen, z.B. mit GM-CSF, verabreicht werden.

Zum Verständnis und zum Anwendungsbereich der Erfindung ist die Anatomie der weiblichen Gebärmutter (*Uterus*) notwendig: Die Gebärmutter besteht aus zwei Abschnitten: dem Gebärmutterkörper (*Corpus uteri*) mit der Gebärmutterhöhle (*Cavum uteri*) und dem Gebärmutterhals (*Cervix uteri*) mit dem Gebärmuttermund (*Portio vaginalis,* meist nur als *Portio* bezeichnet). Der Gebärmutterhals nimmt etwa das untere Drittel der Gebärmutter ein, bei der es sich anatomisch gesehen um ein dickwandiges, muskulöses Hohlorgan handelt, und ragt als Gebärmuttermund (*Portio vaginalis*) in den oberen Teil der Scheide hinein. Der Gebärmutterhals besteht aus Bindegewebe und Muskulatur und weist in Längsrichtung einen Hohlgang auf, den Gebärmutterhalskanal (Cervikalkanal). Dieser ist von einer Schleimhaut ausgekleidet, deren Drüsen einen zähen Schleim bilden. Der Schleim hat die Aufgabe, die Gebärmutterhöhle nach außen zu verschließen und somit vor Keimen aus der Scheide zu schützen. Die Schleimhaut, die den Gebärmutterhals im Bereich des Muttermundes auskleidet, ist flacher als die Schleimhaut im Innern der Gebärmutter. Sie ähnelt dort normaler Schleimhaut, wie sie z.B. in der Mundhöhle vorkommt (Plattenepithel).

Die vorliegende Erfindung dient - wie bereits mehrfach ausgeführt - zur lokalen äußerlichen Anwendung im Bereich der Portio und Zervix uteri, also zur Behandlung der dortigen Schleimhaut. Insofern ist eine klassische Impfung ausgeschlossen und mithin sind Suspensionen, Gele, Salben, Lotionen oder andere im wesentliche liquide Applikationsformen wie Lösungen aller Art in unterschiedlichsten Viskositätsgraden bevorzugt. Im wesentlichen sind aber alle Applikationsformen und galenischen Zusammensetzungen bevorzugt, die zum Auftragen auf die Schleimhaut geeignet sind.

Die folgende Beschreibung betrifft die Art und Weise, auf die die erfindungsgemäßen Viren mit überwiegend üblichen pharmazeutisch unbedenklichen Trägern kombiniert werden, wodurch man zu Dosierungsformen gelangt, die sich für die unterschiedlichen Verabreichungswege, die für einen gegebenen Patienten verwendet werden, und die Erkrankung, krankhafte Störung oder das Leiden, für die bzw. das ein gegebener Patient behandelt wird, eignen. Eine derartige erfindungsgemäße pharmazeutische Zusammensetzung umfasst eine oder mehrere der oben beschriebenen Viren, zusammen mit einem pharmazeutisch unbedenklichen Träger entsprechend den Eigenschaften und dem erwarteten Verhalten solcher dem Fachmann gut bekannten Träger.

Im Rahmen der Erfindung ist daher auch die Verwendung eines Medikaments zur äußerlichen Auftragung auf die Schleimhaut im Bereich der Portio und Zervix uteri beansprucht. Das Medikament ist dazu geeignet, in die obersten Epithelschichten einzudringen. Insofern ist die Verwendung von rekombinaten, genetisch modifizierten in nicht HPV-infizierten Zellen replikationsdefekten E1-deltierten Adenoviren zur Herstellung eines Medikaments zur lokalen äußerlichen Anwendung im Bereich der Portio und Zervix uteri bei menschlichen Patienten hiermit offenbart, wobei das Medikament zur Verabreichung in einer Zieldosis von im Bereich von 1 x 10⁸ bis 1 x10¹² infektiösen Partikeln (Abkürzung: i.p.) über einen Zeitraum von wenigstens 3 Tagen, nämlich am Tag 0, am Tag 3 und am Tag 30 hergerichtet ist.

Ebenfalls offenbart ist die Verwendung einer Zieldosis von 1 x 10¹⁰ infektiösen Partikeln (i.p.) bevorzugt ist. Es können aber auch aufgrund individueller Faktoren Dosisanpassungen denkbar sein, die die angegebene Zieldosis um bis zu zwei Größenordungen unter- oder überschreiten (1 x 10⁸ - 1 x10¹² infektiöse Partikel).

Die Menge an Viren, die mit den Trägermaterialien kombiniert werden kann, um so eine einzelne Dosierungsform zu bilden, hängt von dem behandelten Patienten und dem jeweiligen Verabreichungsweg ab. Es ist jedoch klar, dass ein bestimmtes Dosierungs- und Behandlungsschema für einen bestimmten Patienten von verschiedensten Faktoren, darunter der Wirksamkeit der jeweils verwendeten Verbindung, dem Alter, Körpergewicht, allgemeinem Gesundheitszustand, Geschlecht, der Ernährung, dem Verabreichungszeitpunkt, der Ausscheidungsgeschwindigkeit, der Arzneistoffkombination und dem Ermessen des behandelnden Arztes sowie der Schwere der jeweils behandelten Krankheit abhängt. Die Wirkstoffmenge kann auch von dem Therapeutikum oder Prophylaktikum, das gegebenenfalls mit dem Wirkstoff gemeinsam verabreicht wird, abhängen.

Der Begriff "Träger" umfasst im vorliegenden Zusammenhang unbedenkliche Streckmittel, Exzipienten, Hilfsstoffe, Konstituenten, Lösungsvermittler, viskositätsmodifizierende Mittel, Konservierungsmittel und andere Mittel, die dem Fachmann gut bekannt sind, um der endgültigen pharmazeutischen Zusammensetzung günstige Eigenschaften zu verleihen.

Insbesondere umfasst die bei den erfindungsgemäßen pharmazeutischen Zusammensetzungen verwendeten Träger unterschiedliche Klassen und Arten von Zusatzstoffen, die unabhängig aus den im Wesentlichen in den folgenden Absätzen genannten Gruppen ausgewählt werden.

Antimikrobielle Mittel, darunter Mittel gegen Bakterien, Pilze und Protozoen, werden zugegeben, wenn die pharmazeutische Zusammensetzung topisch auf Hautflächen aufgetragen wird, die wahrscheinlich einer schädigenden Umgebung ausgesetzt waren, oder Abschürfungen oder Schnitte erlitten haben, die die Haut für eine Infektion durch Bakterien, Pilzen oder Protozoen anfällig macht. Antimikrobielle Konservierungsstoffe werden den erfindungsgemäßen pharmazeutischen Zusammensetzungen zugegeben, um diese gegen das Wachstum von möglicherweise schädlichen Mikroorganismen zu schützen, die üblicherweise in die wässrige Phase einwandern, in manchen Fällen jedoch auch in der Ölphase einer Zusammensetzung wachsen können. Es sind daher Konservierungsstoffe erwünscht, die sowohl in wässrigen Medien als auch in Lipiden löslich sind.

Für die topische Anwendung können die pharmazeutischen Zusammensetzungen als geeignete Salbe formuliert werden, die den wirksamen Bestandteil in einem oder mehreren Trägern suspendiert oder gelöst enthält. Die pharmazeutischen Zusammensetzungen können jedoch auch als geeignete Lotion oder Creme, die die wirksamen Bestandteile in einem oder mehreren pharmazeutisch unbedenklichen Trägern suspendiert oder gelöst enthalten, formuliert werden.

Dermatologische Wirkstoffe werden den erfindungsgemäßen pharmazeutischen Zusammensetzungen dort zugegeben, wo diese topisch anzuwenden sind. Dispergier- und Suspendiermittel werden als Hilfsstoffe bei der Herstellung stabiler Formulierungen eingesetzt. Emollientia sind vorzugsweise nichtölige, wasserlösliche Stoffe, die die Haut erweichen und beruhigen, insbesondere Haut, die durch übermäßigen Wasserverlust trocken geworden ist. Solche Stoffe werden bei erfindungsgemäßen pharmazeutischen Zusammensetzungen verwendet, die zur topischen Anwendung bestimmt sind. Emulgatoren, darunter emulgierende und verdickende Mittel und Emulsionshilfsstoffe, werden zur Herstellung von Öl-in-Wasser-Emulsionen verwendet, wenn diese die Grundlage der erfindungsgemäßen pharmazeutischen Zusammensetzungen bilden. Soll die erfindungsgemäße pharmazeutische Zusammensetzung topisch angewendet werden, so können Penetrationsförderer verwendet werden. Verdickungsmittel werden typischerweise bei Formulierungen zur topischen Anwendung verwendet, um diesen die gewünschte Viskosität bzw. die gewünschten Handhabungseigenschaften zu verleihen. Zucker werden häufig verwendet, um den erfindungsgemäßen pharmazeutischen Zusammensetzungen verschiedene erwünschte Eigenschaften zu verleihen und um die erzielten Ergebnisse zu verbessern.

Die Herstellung der erfindungsgemäßen pharmazeutischen Zusammensetzungen erfolgt auf äußerst einfache Art, wie dies dem Durchschnittsfachmann gut bekannt ist. Handelt es sich bei den erfindungsgemäßen pharmazeutischen Zusammensetzungen um einfache wässrige Lösungen bzw. Lösungen in anderen Lösungsmitteln, so werden die verschiedenen Bestandteile der Gesamtzusammensetzung in einer beliebigen praktischen Reihenfolge zusammengegeben, die hauptsächlich von Gründen der Bequemlichkeit bestimmt wird. Diejenigen Bestandteile, die eine geringere Löslichkeit in Wasser, jedoch eine ausreichende Löslichkeit in dem gleichen Hilfslösungsmittel mit Wasser aufweisen, lassen sich alle in diesem Hilfslösungsmittel auflösen, wonach der Wasseranteil des Trägers mit der Hilfslösung versetzt wird, wodurch sich die darin gelösten Stoffe im Wasser lösen. Zur Unterstützung dieses Dispergiervorgangs bzw. Lösungsvorgangs kann ein Tensid eingesetzt werden.

In einer weiteren bevorzugten Ausführungsform der Erfindung liegt das Mittel daher in Form einer Lösung, Emulsion, Suspension, Salbe, eines Balsams, Öls, Gels, Schaums, in flüssiger Form, als thermoreversibles Gel (flüssig anzuwenden), in Form eines wirkstoffhaltigen Tampons und/oder in Form einer Creme vor. Es war völlig überraschend, dass die Vorteile des erfindungsgemäßen Mittels noch einmal verbessert werden können, indem es in die genannten galenischen Formen eingebracht wird. Dem Fachmann sind weitere Formulierungskonzepte für das Einbringen der erfindungsgemäßen Mittel in Trägersubstanzen bekannt wie z. B. Emulsionen, oder andere Produkte zur dermalen Applikation wie z. B. flüssige Formen, die bevorzugt wasserfrei oder wasserhaltig sein können, wobei die wasserhaltigen erfindungsgemäß in Einphasensysteme und in Mehrphasensysteme unterschieden werden können. Weiterhin können halbfeste Formen, die wasserfrei oder wasserhaltig sind, eingesetzt werden, wobei wiederum eine Einteilung in Einphasensysteme und Mehrphasensysteme bei den wasserhaltigen halbfesten Formen möglich ist. Weiterhin können bevorzugt feste Formen eingesetzt werden, die lipophil oder hydrophil sind. Beispiele für solche Formen sind neben den genannten z. B. Fettsalben, Schäume, Gelcremen, Hydrodispersionsgele, dünnflüssige Emulsionen, Lotionen, Salben, Sprays und Cremes.

Das erfindungsgemäße Mittel bzw. die pharmazeutische Zusammensetzung, resp. Das daraus resultierende Arzneimittel, weist ein Galenik auf, die das Auftragen auf Schleimhäute ermöglicht.

Dem Fachmann bekannt sind dabei unterschiedliche Zusammensetzungen im Hinblick auf die Viskosität in niedrigviskose und in hochviskose. Nanoemulsionen oder Öle bzw. Oleogele weisen eher eine niedrige Viskosität auf, wohingegen Hydrogele oder Hydrocremen bzw. O/W-Emulsionen oder W/O-Emulsionen eine hohe Viskosität aufweisen. Wenn flüssige Applikationsformen zum Einsatz kommen, können diese - wie oben ausgeführt - in wasserfreie und wasserhaltige Systeme unterteilt werden. Bei den wasserfreien Systemen sind insbesondere apolare Systeme, polare Systeme ohne Emulgatoren und polare Systeme mit Emulgatoren bevorzugt. Bei den wasserhaltigen Systeme sind einphasige Systeme wie Lösungen und Mikroemulsionen bevorzugt, bei den mehrphasigen Systemen sind multiple Emulsionen, W/O-Emulsionen oder O/W-Emulsionen bevorzugt. Bei den Fest/Flüssig-Systemen sind bevorzugte Formen die Suspensionen oder die Flüssig/Fest/Flüssig-Systeme wie Suspensions-/Emulsionssysteme. Dem Fachmann sind verschiedene Möglichkeiten bekannt, derartige pharmazeutische Träger bereitzustellen. Bei den O/W-Emulsionen sind bevorzugte galenische Leitsubstanzen O/W-Emulgatoren, W/O-Emulgatoren, flüssige hydrophile Bestandteile und flüssige lipophile Bestandteile. Bei den W/O-Emulsionen sind bevorzugte galenische Leitsubstanzen W/O-Emulgatoren, O/W-Emulgatoren, flüssige und halbfeste lipophile Bestandteile, Gelbildner, flüssige hydrophile Bestandteile und/oder Salze.

Besonders vorteilhaft können auch Nanotransportsysteme mit dendritischer Architektur verwendet werden, wie sie in der DE 10 2004 039 875 offenbart sind. Diese ist in den Offenbarungsgehalt der vorliegenden anmeldungsgemäßen Lehre mit aufgenommen. Aus dem Stand der Technik sind verschiedene Transportsysteme bekannt wie beispielsweise Liposomen, Polymermizellen, Poly-merkonjugte oder einfache dendritische Kern-Schale-Architekturen. Polymermizellen sind physikalische Aggregate ambiphiler Makromoleküle, die sich in Wasser spontan durch Selbstorganisation bilden können. Meistens ist der innere Block unpolar oder ionisch und der äußere Block, der den Kern durch sterische Stabilisierung schützt, polar. Sie werden häufig zur Solubilisierung unpolarer Wirkstoffe oder Wirkstoffkombinationen mit begrenzter Löslichkeit in Wasser bzw. zum Transport von Oligonukleotiden verwendet. Derartige dermale Transportsysteme können vorteilhafterweise eingesetzt werden, um eine schnelle Passage des erfindungsgemäßen Mittels in Tumoren zu ermöglichen.

Nanotransportsysteme mit einfacher dendritischer Kern-Schale Architektur ermöglichen ebenfalls gezielte Wirkstofftransporte. Im Unterschied zu physikalischen Aggregaten ambiphiler Moleküle können durch die kovalente Modifizierung dendritischer Makromoleküle mit entsprechender Schale stabile mizellartige Strukturen erhalten werden, welche für die Verkapselung von Arzneistoffen geeignet sind. Besonders vorteilhaft können Nanotransportsysteme eingesetzt werden, welche mindestens aus einem dendritischen Kern und mindestens zwei Schalen bestehen. Vorzugsweise weisen die Schalen unterschiedliche Polaritäten auf, somit wird ein Polaritätsgradient erreicht, um welchen sowohl unpolare als auch polare Wirkstoffe oder Kombinationen von Wirkstoffen eingeschlossen werden können. Die vorteilhaften Nanotransportsysteme weisen daher vorteilhafterweise eine mehrschalige unimolekulare Struktur auf. Durch die Kombination der verschiedenen Schalen ist es sehr gut möglich, immer wieder neue auf den Wirkstoff und dessen Verwendung abgestimmte Nanotransportsysteme zu kreieren. Für ein derartiges Nanotransportsystem können sowohl Dendrimere als auch hyperverzweigte Polymere eingesetzt werden. Vorteilhaft ist somit auch der Einsatz von hyperverzweigten Polymeren, wodurch vorteilhafterweise der Syntheseaufwand und auch die Kosten herabgesetzt werden können. In einer bevorzugten Ausführungsform besteht der o. g. dendritische Kern aus Polyglycerin, Polyamid, Polyamin, Polyether oder Polyester. Diese Verbindungen können innerhalb der dendritischen Architektur auch weiter modifiziert werden. Der dendritische Kern kann somit gemäß seiner Modifikation polarisiert sein. Es war völlig überraschend, dass derartige Mittel besonders gut bei der Applikation durch die Haut eingesetzt werden können. Sie sind ca. viermal so gut geeignet, Partikel in die Haut zu applizieren, wie anderen Lipid-Nanopartikel.

Offenbart wird auch erfindungsgemäße Applikatoren, die unter Vermeidung einer Blutung zur Verabreichung des erfindungsgemäßen Mittels bzw. der pharmazeutische Zusammensetzung bzw. des Arzneimittels bevorzugt sind. Hierbei ist besonders bevorzugt ein Applikator, umfassend eine Injektionseinrichtung, die unter Vermeidung einer lokalen Blutung dazu geeignet ist, in die obersten Epithelschichten einzudringen. Um den lokalen Viruseintrag in das Epithel der Portio zu verbesseren, sind daher solche Applikatoren bevorzugt, die eine modifizierte Oberflächenstruktur aufweisen, beispielsweise winzig kleine Spitzen tragen, die jedoch so kurz sind, dass sie nur kleine Löcher in das Epitehel stanzen, ohne eine Blutung zu verursachen.

Die Erfindung wird nun anhand von Beispielen und Figuren näher beschrieben; es zeigt:
- **Fig. 1**: Schematisch die im Onkoprotein HPV18-E6 vorhandenen Proteinbindungsdomänen (Funktionsdomänen) und deren Lokalisierung;
- **Fig. 2**: Nukleinsäuresequenz des Vakzinierungsgens p14;
- **Fig. 3**: Aminosäuresequenz des Vakzinierungsgens p14;
- **Fig. 4**: die Präzipitation von LxCxE-tragenden GSTp14-Fusionskonstrukten nach Inkubation mit Kernextrakten;
- **Fig. 5**: Abbildung zum Nachweis des funktionellen Synergismus von HPV-Onkogen exprimierenden Zellen und E1-deletierten A-denoviren durch Messung der Reportergenexpression in HPV-Onkogen positiven und HPV-Onkogen negativen Zellen nach adenoviralem Gentransfer eines Reportergens (Messung der Stärke der relativen gfp-Expression pro Zelle nach Infektion mit Ad-gfp);
- **Fig. 6**: Abbildung zum Nachweis des funktionellen Synergismus von HPV-Onkogen exprimierenden Zellen und E1-deletierten A-denoviren durch Messung der adenoviralen Genomkopien in HPV-Onkogen positiven und HPV-Onkogen negativen Zellen nach adenoviralem Gentransfer eines Reportergens (Messung der Kopien adenoviraler DNA / ß-Aktin).
- **Fig. 7, 7a**: Direkter Nachweis der Infizierbarkeit humaner Gebärmutterhalskanalepithelien mit einem Reportergen-tragenden Adenovirus (Ad-lacZ).

### Ausführungsbeispiele

### 1. Vakzinierungsgen

Das Vakzinierungsgen entspricht demjenigen, welches bereits in der WO 2009/106362 (dort Beispiel 1: "Klonierung eines rekombinanten therapeutischen Vakzinierungsgens ohne Transformations-assoziierte Peptidmotive") sowie in Hoffmann et al. (J. Immunother 2010; 33: 136-145) beschrieben ist. Hierauf, sowie auf den gesamten Offenbarungsgehalt der WO 2009/106362 und Hoffmann et al. (J. Immunother 2010; 33: 136-145) wird Bezug genommen und als Referenz in die vorliegende Anmeldung inkorporiert.

Das Vakzinierungsgen (p14) wurde durch Fusion von 14 Bruchstücken der beiden HPV-On-kogene E6 und E7 der *high risk* HPV 16 und HPV 18 kloniert und ist in der Lage eine zelluläre Immunität gegen Onkogene der Serotypen HPV16 und HPV18, sowie sequenzverwandter weiterer HPV Serotypen zu induzieren. Es besitzt keine transformierenden Eigenschaften mehr. Das Vakzinierungsgen wurde in einen E1-deletierten Adenovirus einkloniert und sein therapeutisches Potential in Mäusen untersucht. Die einmalige intramuskuläre Applikation von Ad-p14 in Mäusen (C57BL6) in einer Dosis von 1x10e10 i.p. induziert eine Immunantwort, die nicht nur das Anwachsen von HPV-Onkogen- exprimierenden Tumorzellen zuverlässig verhindert, sondern auch bereits etablierte Tumore zur sicheren und dauerhaften Abheilung bringt.

### Im Einzelnen:

### 1a. Identifizierung von Transformations-assoziierten Peptidmotiven in HPV-Onkoproteinen

Ein von einem Onkogen kodiertes Protein vermittelt seine onkogene Wirkung oftmals, indem es an körpereigene zelluläre Proteine bindet und diese in ihrer physiologischen Wirkung beeinflusst bzw. sie einer schnellen Degradation zuführt.

Meist handelt es sich bei den betroffenen zellulären Proteinen um Moleküle, die eine wichtige Rolle bei der Steuerung des Zellzyklus spielen. Die Wechselwirkung zwischen Onkogen und Zellzyklusprotein findet über kurze, manchmal nur wenige Aminosäuren umfassende Peptidmotive statt. Tabelle 1 zeigt die wichtigsten Proteinbindungsmotive von HPV16 E6- und E7-Onkogenen, die durch Deletionsstudien identifiziert worden sind.

**Tabelle 1:**

| Sequenzmotive aus HPV16-E6 und -E7, die an der Vermittlung transformierender Eigenschaften beteiligt sind ("AS" steht für Aminosäure). | | | |
|---|---|---|---|
| **Proteinmotiv** | **AS-Position in E6 HPV16** | **Funktion** | **Literatur** |
| **-MFQ-** | 8-10 | Degradation von p53 | Klingelhutz AJ et al., Nature. 1996;380 (6569): 79-82 |
| | | | |
| | | | Huibregtse JM et al., Mol Cell Biol. 1993;13 (8): 4918-4927 |
| | | | |
| **-CxxC-** | 37-40 | Ausbildung zweier Zinkfingerformationen | Kanda T et al., Virology. 1991;182 (2): 723-731 |
| | | | |
| | | | Sherman L et al., J Virol. 1996; 70 (5): 3269-3279 |
| | 70-73 | | |
| | 110-113 | | |
| | 43-146 | | |
| | | | |
| **-CPEE-** | 118-121 | Telomeraseaktivierung | Klingelhutz AJ et al., Nature. 1996;380 (6569): 79-82 |
| | | | |
| **-RRETQL(V)** | 153-158 | Bindung von PDZ-Domänen enthaltenden Proteinen | Kiyono T et al., Proc Natl Acad Sci USA 1997; 94 (21): 11612-11616 |
| | | | |
| | | | Brehm A et al., Nature 1998; 391 (6667): 597-601 |

| | **AS-Position In E7 HPV16** | | |
|---|---|---|---|
| **-LxCxE-** | 22-26 | vermittelt Rb-Bindung und aktiviert Histondeacetylase, fördert die Zellproliferation | el Deiry WS et al., Cell 1993; 75 (4): 817-825 |
| | | | |
| **-CxxC** | 58-61 | Ausbildung einer Zinkfingerformation | |
| | 91-94 | | |
| **-EDLL-** | 80-83 | Verändert die Funktion von Zellzyklusproteinen (p21waf-1, p27Kip1, S4, M2-PK) und stimuliert die aerobe Glycolyse | Polyak K et al., Cell 1994; 78 (1): 59-66 |
| | | | |
| | | | Zwerschke W et al., Proc Natl Acad Sci U S A 1999; 96 (4): 1291-1296 |
| | | | |
| | | | Shimizu J et al., Nat Immunol. 2002; 3 (2): 135-142 |

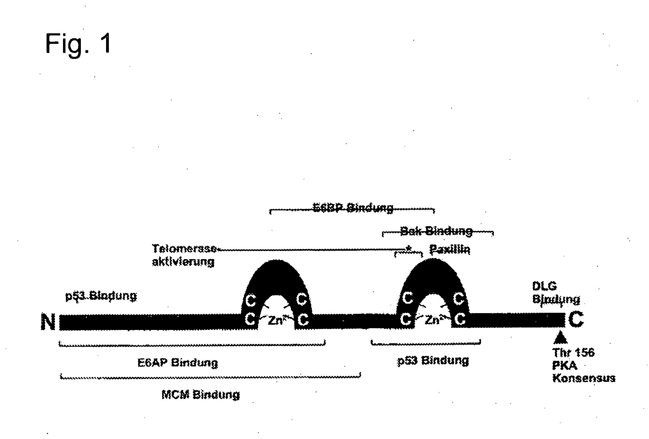

**Fig. 1** zeigt schematisch die im Onkoprotein HPV18-E6 vorhandenen Proteinbindungsdomänen und deren Lokalisierung.

In den in der Vergangenheit hergestellten therapeutischen HPV-Vakzinen sind diese Proteinbindungsmotive nahezu komplett erhalten. Selbst das von Öhlschläger und Kollegen (Ohlschlager P et al., Vaccine. 2006; 24 (15): 2880-2893) vorgestellte Konzept der Neuordnung von Bruchstücken verändert zwar die Position des Bindungsmotives im Vakzinierungsgen im Vergleich zum Wildtyponkogen, es zerstört aber nicht die Integrität des Bindungsmotives. Bindungsstudien mit synthetischen Peptiden wie LxCxE (AS 22-26 in HPV16-E7) oder das PDZ-Domänen bindende RRETQL am C-Terminus von E6 (AS 153-158 in HPV16-E6) dokumentieren die hohe Affinität dieser Peptidmotive zu Zellzyklusproteinen wie dem Rb/Histonedeacetylasekomplex oder verschiedenen PDZ-Domänen-enthaltenden Tumorsuppressorproteine wie MAGI-1 oder SAP97/dlg (P.L. Triozzi et al., J Immunother 28 (2005), pp. 382-38826; B. Klencke et al., Clin Cancer Res 8 (2002), pp. 1028-1037; J. Tartaglia et al., Virology 188 (1992), pp. 217-232).

### 1b. Klonierung des Vakzinierungsgens

Zur Konstruktion des therapeutischen Vakzinierungsgens (p14) wurden 14 DNA-Fragmente aus HPV16 und HPV18 E6- und E7-Onkogenen ausgewählt und spiegelbildlich zur Anordnung in den Wildtypgenen unter Berücksichtigung des korrekten Leserahmens kloniert. Jedes der 14 DNA-Fragmente kodiert für 21-36 Aminosäuren (Tabelle 2). Die adenoviralen Basisvektoren und die Klonierungsstrategie wurden wie in Schwieger et al. (Carcinogenesis 2001 Sep; 22 (9):1385-9250) beschrieben verwendet.

**Tabelle 2:**

| Sequenzfragmente aus HPV16 und HPV18 E6 und E7 Onkogenen, die zur Klonierung des Vakzinierungsgen p14 ausgewählt wurden. | | | |
|---|---|---|---|
| | *Position in HPV16 E7* | | *Position in p14* |
| 1) | aa 1-21: | MHGDTPTLHEYMLDLQPETTD | aa 55-75 |
| 2) | aa 27-57: | QLNDSSEEEDEIDGPAGQAEPDRAHYNIVTF | aa 22-52 |
| 3) | aa 62-79: | DSTLRLCVQSTHVDIRTL | aa 2-19 |

| | *Position in HPV18 E7* | | |
|---|---|---|---|
| 4) | aa 1-24: | MHGPKATLQDIVLHLEPQNEIPVD | aa 135-158 |
| 5) | aa 30-62: | QLSDSEEENDEIDGVNHQHLPARRAEPQRHTML | aa 100-132 |
| 6) | aa 67-86: | KCEARIELVVESSADDLRAF | aa 78-97 |

| | *Position in HPV18 E7* | | |
|---|---|---|---|
| 7) | *aa 12-36:* | PQERPRKLPQLCTELQTTIHDIILE | aa 253-277 |
| 8) | *aa 41-69:* | KQQLLRREVYDFAFRDLCIVYRDGNPYAV | aa 222-250 |
| 9) | *aa 74-109:* | LKFYSKISEYRHYCYSLYGTTLEQQYNKPLCDLLIR | aa 184-219 |
| 10) | *aa 122-142:* | KQRHLDKKQRFHNIRGRWTGR | aa 161-181 |

| | *Position in HPV18 E7* | | |
|---|---|---|---|
| 11) | *aa 7-31:* | PTRRPYKLPDLCTELNTSLQDIEIT | aa 367-391 |
| 12) | *aa36-64:* | KTVLELTEVFEFAFKDLFVVYRDSIPHAA | aa336-364 |
| 13) | *aa 69-104:* | IDFYSRIRELRHYSDSVYGDTLEKLTNTGLYNLLIR | aa 298-333 |
| 14) | *aa 122-137:* | NEKRRFHKIAGHYRGQ | aa 280-295 |

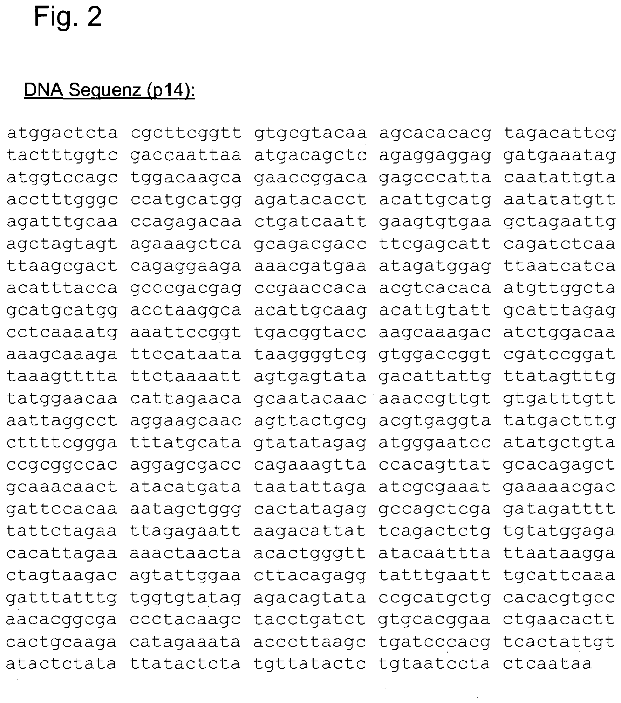

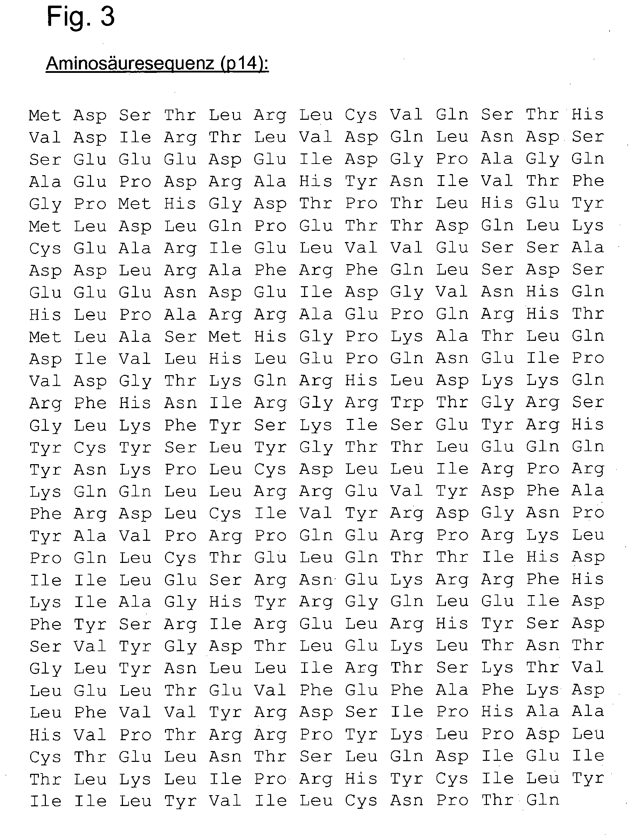

Das gesamte Vakzinierungsgen codiert für ein 415 Aminosäuren (1248 bp). Die Nukleinsäure-und Aminosäuresequenz von p14 sind in **Fig. 2** **und** **3** (SEQ ID No. 1 und 2) gezeigt. Die Expression erfolgt unter Kontrolle eines CMV-Promoter und eines CMV-Polyadenylierungssignals. Ein Ausschluss potentiell gefährlicher Sequenzmotive führt zwangsläufig auch zum Verlust von potentiellen T-Zellepitopen. Bei der zur Klonierung des Vakzinierungsgens ausgewählten Onkogenfragmente wurde versucht, den Verlust an therapeutisch relevanten T-Zellepitopen gering zu halten, indem die eingefügten Deletionen exakt auf den Umfang der identifizierten Bindungsmotive beschränkt blieben. Eine Analyse des hergestellten Vakzinierungsgens bezüglich der Zahl der verbliebenen T-Zellepitope zeigt, dass 7 von 8 der zu den 4 häufigsten MHC-Klasse I Molekülen am höchsten affinen E6-Epitope von HPV 16 und 18 noch im Gen kodiert werden (Tabelle 3).

**Tabelle 3**

| Die Tabelle zeigt die An- (1) bzw. Abwesenheit (0) der höchstaffinen T-Zellepitope der p14-Vakzine im Bezug auf die 4 in der Bevölkerung häufigsten MHC-Klasse I Allele. Trotz der aus Gründen der biologischen Sicherheit durchgeführten Sequenzrestriktionen kodiert die vorgestellte p14-Vakzine noch für alle 5 der höchstaffinen HLA-A0201 Epitope (etwa 50 % der Bevölkerung tragen diesen MHC-Klasse 1 Locus) sowie über die 4 häufigsten MHC-Klassen hinweg 7 von 8 der höchstaffinen Epitope. | | |
|---|---|---|
| | ***HPV16-E6*** | ***HPV18-E6*** |
| ***A 0201*** | 1-1-1-1-1 (5/5) | 1-1-1-0-0 (3/5) |
| ***A 2402*** | 1-1-0-1-1 (4/5) | 1-0-0-0-1 (2/5) |
| ***A 0101*** | 1-0-1-0-1 (3/5) | 0-1-1-0-0 (2/5) |
| ***A 0301*** | 1-0-0-1-0 (2/5) | 1-0-0-0-1 (2/5) |

Die durch Fragmentierung und Ausschluss von Peptidbindungsmotiven vollzogene vollständige Neutralisierung des transformierenden Potenzials der Wildtyponkogene wurde in Transformationsassays geprüft (Schwieger et al., Carcinogenesis 2001 Sep; 22 (9):1385-9250). Es konnte gezeigt werden, dass das rekombinante p14-Vakzinierungsgen im Gegensatz zu den Wildtyponkogenen keinerlei transformierende Aktivität mehr besitzt (Tabelle 4).

**Tabelle 4:**

| Der p14-Vektor zeigt im Vergleich zu den Ausgangskonstrukten keinerlei transformierende Aktivität mehr. | | | |
|---|---|---|---|
| ***Vektor*** | ***Kolonien*** | ***Transfektionseffizienz (1000 Zellen)*** | ***Transformations-frequenz*** |
| **p*CMV-p14*** | 0 | 21 % | 0 % |
| **p*HPV16E6*/*E7*** | *138* | *19 %* | *66 %* |
| **p*HPV18E6*/*E7*** | 21 | 38 % | 5,5 % |
| **p*ras*** | 121 | 39 % | 31 % |
| **p*CMVSL* (mock)** | 0 | 17 % | 0 % |

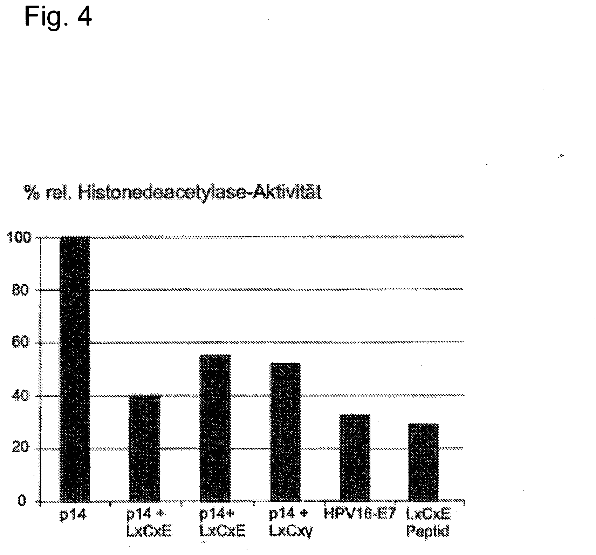

**Fig. 4** zeigt, dass die Präzipitation von LxCxE-tragenden GSTp14-Fusionskonstrukten nach Inkubation mit Kernextrakten zu einer Abnahme der Histonedeacetylaseaktivität im Überstand führt. Der Effekt ist unabhängig von der Position des LxCxE Motivs innerhalb des p14-Proteins (α,β,γ).

Somit verleiht das Wiedereinfügen des LxCxE Motivs dem Expressionsprodukt im Vergleich zum p14-Ausgangskonstrukt eine erhöhte Affinität zu Rb-Histonedeacetylasekomplexen. Dieser Effekt ist relativ positionsunabhängig und macht damit deutlich, welche Gefahr von der Anwesenheit dieses oder ähnlicher Bindungsmotive im Vakzinierungsgen ausgeht.

Mit der direkten Kompetition zwischen dem Retinoblastom-Tumorsuppressorgen (Rb) und LxCxE ist eine Störung der Proliferationskontrolle verbunden, die mit einer erhöhten Proliferationsneigung verbunden ist. Auf diese Weise wird deutlich, dass unerwünschte Wechselwirkungen zwischen Vakzine und zellulären Proteinen nur durch konsequente Eliminierung aller potentiell proteinbindenden Bereiche aus der Vakzine gewährleistet werden kann. In dem vorgestellten Vakzinierungsgen p14 sind alle in der Tabelle 1 enthaltenen Sequenzmotive aus HPV16-E6 und E7 bzw. die zu ihnen korrespondierenden Sequenzen aus HPV18-E6 und E7 nicht mehr präsent. Es zeigt sich, dass es dennoch möglich ist ein Vakzinierungsgen zu klonieren, das ein eindrucksvolles immuntherapeutisches Potenzial besitzt und das gleichzeitig ein Maximum an biologischer Sicherheit gewährleistet. Die vorgestellte DNA-Vakzine ist das zur Zeit sicherste und gleichzeitig leistungsfähigste Konstrukt seiner Art.

### 2. Verstärkung der Expression therapeutischer Gene durch selektive adenovirale in HPV-Onkogen exprimierenden Zelllinien

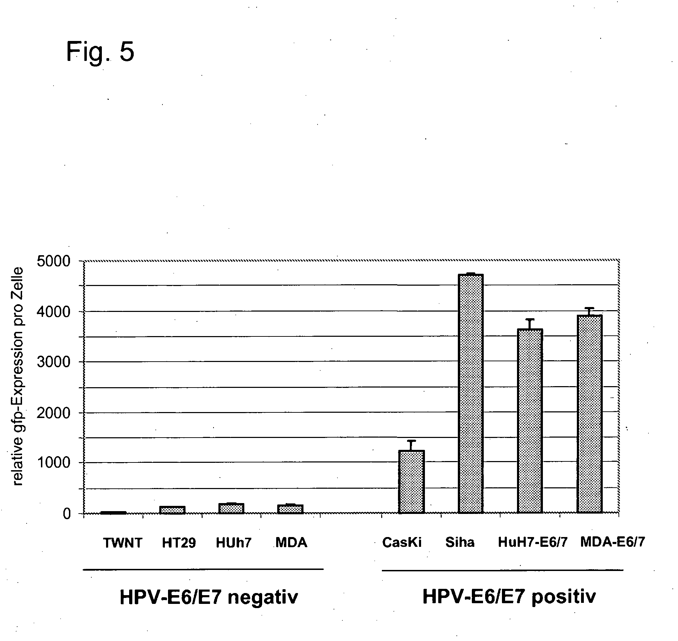

Nachdem in früheren Untersuchungen die biologische Sicherheit der Vakzine und ihre prinzipielle Eignung zur Induktion einer spezifischen gegen HPV-Onkogene gerichtete T-Zellantwort untersucht wurde, soll in weiteren Untersuchungen ihre Eignung als äußerlich (mukosal) applizierbare Vakzine gezeigt werden. Des weiteren soll insbesondere ihre Fähigkeit, sich in HPV-Onkogen exprimierenden Zellen zu replizieren gezeigt werden. Um den Mechanismus der Unterstützung einer genomischen Replikation E1-deletierter Adenoviren durch HPV-On-kogen-exprimierende Zellen zu untersuchen, wurden HPV-E6 und E7 positive Zellinien (CasKi, Siha, Huh7-E6/7, MDA-MB468-E6/7) und HPV-negative Zelllinien (TWNT, HT1080, Huh7, MDA-MB468) mit einem ein Reportergen-tragenden E1-deletierten Adenovirus (Ad-gfp) in einer MOI (multiplicity of infection) von 10 infiziert und die gfp-Expression pro Zelle 48 Stunden nach Infektion ermittelt. Bei vergleichbarer Transfektionseffizienz liegt die gfp-Expression in HPV-Onkogen-positiven Zelllininen um Faktor 10 - 50 höher als in HPV-negativen Zellinien (**Fig. 5**). Die Infektion einer HPV-Onkogen (E6 und E7) exprimierenden Zelle mit einem E1-deletierten Adenovirus (Ad-gfp) führt zu einer 10 - 50 fachen Verstärkung der Expression des adenoviralen Transgens (gfp). Die HPV-Onkogene sind in der Lage die adenovirale Replikationsmaschinerie zu aktivieren und erhöhen auf diese Weise die Kopienzahl der adenoviralen Genome, wodurch die Expressionstärke der kodierenden Transgene erhöht wird. Es konnte also der Nachweis des funktionellen Synergismus von HPV-Onkogen exprimierenden Zellen und E1-deletierten Adenoviren durch Messung der Reportergenexpression in HPV-Onkogen positiven und HPV-Onkogen negativen Zellen nach adenoviralem Gentransfer eines Reportergens geführt werden.

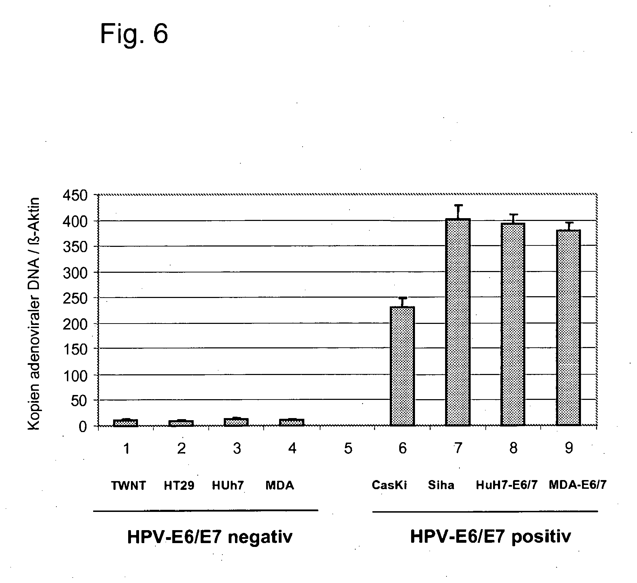

Die Bestimmung der Anzahl der genomischen adenoviralen Kopien bezogen auf die Anzahl von ß-Actinkopien unter Berücksichtigung der Transfektionseffizienz durch quantitative PCR zeigt, dass ein linearer Zusammenhang zwischen der Expressionshöhe und der Anzahl der pro Zelle vorhandenen Kopien besteht (siehe **Fig. 6**). 48 Stunden nach Infektion einer HPV-Onkogen positiven Zelle konnten bis zu über 400 genomische Kopien des adenoviralen Genoms pro ß-Actinkopie gefunden werden, während in HPV negativen Zellen maximal bis 20 Kopien nachgewiesen werden konnten. Fig. 6 zeigt daher die Anzahl adenoviraler Kopien 48 Stunden nach Infektion HPV-Onkogen positiver und HPV-Onkogen negativer Zellinien. Es ist erkennbar, dass die Infektion einer HPV-Onogen-exprimierenden Zelle zu einer Aktivierung der adenoviralen Replikationsmaschinerie führt und auf diese Weise die Transgenexpression steigert..

### 3. Tierversuch

Es wurde ein Tierversuch zum Nachweis einer spezifischen T-Zellaktivierung durchgeführt, wobei durch äußerliche, mukosale (sublinguale) Applikation von Ad-p14 in Mäusen (C57BL6) anhand der spezifischen Wirkung das Anwachsen HPV-Onkogen (HPV16 E6/E7) exprimierender Tumore (C3) verhindert werden konnte. In Anbetracht der Tatsache, dass bei Tieren, insbesondere Mäusen eine lokale äußerliche Anwendung im Bereich des Gebärmutterhalses unter anatomischen und praktikablen Gesichtspunkten nicht in Betracht kommt, dient der vorliegende Versuch der grundsätzlichen Anwendbarkeit und der Funktionsfähigkeit des erfindungsgemäßen Mittels.

Untersucht wurde insoweit die Wirksamkeit einer mukosalen Vakzinierung an Mäusen (C57BI6) mit dem HPV-Onkogenepitop-exprimierenden Adenovirus Ad-p14, im Vergleich zu einem Kontrolladenovirus (Ad-lacZ) und der Gabe von Puffer allein (Mock). Dieser Versuch ist insbesondere im Hinblick auf die Wirksamkeit einer Schleimhaut-Appllikation ("mukosal") der Erfindung zu sehen. Denn die bereits oben beschriebene Vakzinierung im Wege einer intramuskulären Injektion (vgl. auch WO 2009/106362 A1 und Hoffmann et al., J. Immunother 2010; 33: 136-145) ist nicht gleichzusetzen mit einer Anwendbarkeit im Bereich der Schleimhaut.

Um die prinzipielle Wirksamkeit einer mukosalen Applikation (sublingual) zu zeigen, wurde narkotisierten Mäusen (C57BL6) 20 µl einer Ad-p14 haltigen Virussuspension (1 x 10¹⁰ i.p) vorsichtig mit der Pipette unter die Zunge appliziert (sublingual). In den Kontrollgruppen wurde die gleiche Menge eines Kontrollvirus (Ad-lacZ) bzw. nur Puffer (Mock) appliziert. 10 Tage danach wurde 1 x 10⁶ C3-Tumorzellen subkutan unter die rechte Flanke appliziert (gespritzt) und das Tumorwachstum beobachtet. Während in der Kontrollgruppe (nur Puffer) alle behandelten Tiere (10/10) Tumorwachstum zeigten (100%), wuchsen in der zweiten Kontrollgruppe (Ad-lacZ) 9 von 10 Tumoren (90%) an. In der zuvor mit Ad-p14 behandelten Gruppe von Mäusen wuchs jedoch nur in einer der so behandelten Tiere ein Tumor an (10%), was die Durchführbarkeit einer mukosalen Vakzinierung deutlich macht (vgl. Tabelle 5).

**Tabelle 5**

| Anzahl der Tiere mit und ohne Tumorwachstum | |
|---|---|
| Mock | 10/10 |
| Ad-lacz | 9/10 |
| Ad-p14 | 1/10 |

Die Ergebnisse gem. Tabelle 5 verdeutlichen den grundsätzlichen Erfolg des Tierversuchs, wonach ein Tumorwachstum verhindert werden konnte mittels der sublingualen Applikation des erfindungsgemäßen Mittels. Eine vorangegangene Vakzinierung mit Ad-p14 verhindert also zuverlässig das Auswachsen der Tumore.

### 3. Humanversuch

Um die Infizierbarkeit von Epithelien des menschlichen Gebärmutterhalses deutlich zu machen wurde ein kleines noch vitales Gewebestück (eines Operations-Präparates nach Hysterektomie), nämlich ein 3 mm durchmessender Gewebezylinder aus Gewebe eines humanen Uterus (Zervixkanal) einer Patientin isoliert und innerhalb von 60 Minuten nach operativer Entnahme mit einem ein Reportergen tragenden rekombinanten Adenovirus (Ad-lacZ) in einer Konzentration von 2 x 10⁹ infektiösen Partikeln (i.p)/ml für 30 Minuten infiziert und anschließend für 48 Stunden in begastem Kulturmedium bei 37°C kultiviert.

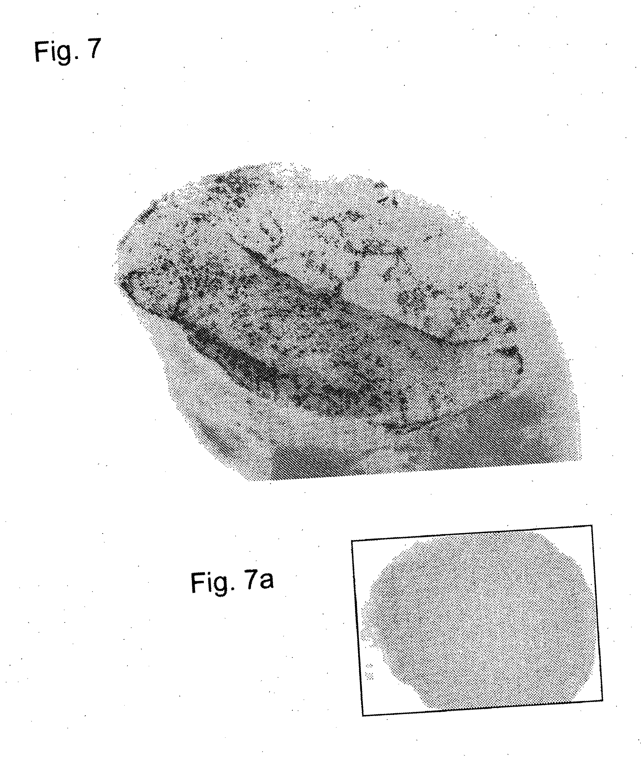

Anschließend wurde nach kurzer Fixierung in Formalin eine X-Gal Färbung zur Darstellung der intrazellulären Reportergenaktivität (Expression des Transgens LacZ für ß-Galactosidase (ß-gal)) durchgeführt. Das histologische Bild in Fig. 7 zeigt die gute Infizierbarkeit humaner Zervxiepithelien anhand der charakteristischen Färbungen. Dies wird insbesondere deutlich im Vergleich mit einem nicht-transfizierten Kontrollgewebe (vgl. **Fig. 7a**), welches keinerlei lacZ-Expression zeigt.

## Patentansprüche

1. Mittel zur Verwendung als Medikament zur Behandlung von Zervixdysplasien, umfassend einen rekombinanten, genetisch modifizierten in nicht-HPV-infizierten Zellen replikationsdefekten E1-deletierten Adenovirus, wobei das Mittel lokal und äußerlich im Bereich der Portio und Zervix uteri verabreicht wird und der E1-deletierte Adenovirus ein oder mehrere Papillomavirus-Antigenepitope repräsentierende Vakzinierungsgene umfasst, deren Expression geeignet ist eine zelluläre oder humorale Immunantwort gegen HPV-infizierte oder HPV-Onkogen exprimierende Zellen zu erzeugen..

2. Mittel zur Verwendung nach Anspruch 1, wobei die Vakzinierungsgene eine Immunantwort gegen die Human Papilloma Viren (HPV) 16, 18, 25, 31, 33, 35, 39, 45, 51, 52, 53, 56, 58, 59, 66, 68, 73 oder 82 hervorrufen, besonders bevorzugt gegen HPV16 und HPV18.

3. Mittel zur Verwendung nach Anspruch 2, umfassend ein durch Fusion von Bruchstücken der HPV-Onkogene E6 und E7 der *high risk* HPV16 und HPV18 hergestelltes Vakzinierungsgen.

4. Mittel zur Verwendung nach Anspruch 3, wobei das Vakzinierungsgen die Sequenz entsprechend SEQ ID No. 1 aufweist.

5. Mittel zur Verwendung nach Anspruch 3, wobei das Vakzinierungsgen für die Proteinsequenz gern. SEQ ID No. 2 kodiert.

6. Mittel zur Verwendung nach einem oder mehreren der vorhergehenden Ansprüche, wobei der E1-deletierte Adenovirus ein oder mehrere immunmodulatorische Gene umfasst, die geeignet sind eine verstärkte Immunantwort gegen HPV-infizierte Zellen zu erzeugen.

7. Mittel zur Verwendung nach Anspruch 6, wobei das immunmodulatorische Gen ein inflammatorisches Interleukin ist.

8. Pharmazeutische Zusammensetzung zur Verwendung als Medikament von Zervixdysplasien zur lokalen äußerlichen Anwendung im Bereich der Portio und Zervix uteri, umfassend wenigstens das Mittel nach einem oder mehreren der vorhergehenden Ansprüchen, sowie galenische Zusatzstoffe, die zur äußerlichen lokalen Anwendung im Bereich der Portio und Zervix uteri geeignet sind.

9. Pharmazeutische Zusammensetzung zur Verwendung nach Anspruch 8, umfassend zumindest eine weitere pharmazeutisch wirksame Substanz.

10. Pharmazeutische Zusammensetzung zur Verwendung nach Anspruch 8 oder 9, wobei diese als Suspension, Gel, Salbe, Lotion oder als Lösung vorliegt.

11. Arzneimittel zur Verwendung bei der Behandlung von Zervixdysplasien zur lokalen äußerlichen Anwendung im Bereich der Portio und Zervix uteri, umfassend eine pharmazeutische Zusammensetzung nach Anspruch 8 bis 10.

## Claims

1. An agent for use as a medicament for the treatment of cervical dysplasia, comprising a recombinant genetically modified replication-defective E-deleted adenovirus in non-HPV-infected cells, wherein the agent is administered locally and externally in the region of the portio and cervical uteri and the E1-deleted adenovirus comprises one or more papillomavirus antigen epitope representing vaccination genes, the expression of which is suitable to generate a cellular or humoral immune response against HPV- infected or HPV-oncogene expressing cells.

2. The agent for use according to claim 1, wherein the vaccination gene causes an immune response against human papilloma viruses (HPV) 16, 18, 25, 31, 33, 35, 39, 45, 51, 52, 53, 56, 58, 59, 66, 68, 73 or 82, more preferably against HPV16 and HPV18.

3. The agent for use according to claim 2, comprising a vaccination gene produced by fusing fragments of the HPV oncogenes E6 and E7 of the *high-risk* HPV 16 and HPV 18.

4. The agent for use according to claim 5, wherein the vaccination gene exhibits the sequence according to SEQ ID No. 1.

5. The agent for use according to claim 5, wherein the vaccination gene encodes the protein sequence according to SEQ ID No. 2.

6. The agent for use according to one or more of the preceding claims, wherein the E1-deleted adenovirus comprises one or more immunomodulatory genes that are suitable for producing an enhanced immune response against HPV-infected cells.

7. An agent for use according to claim 6, wherein the immunomodulatory gene is an inflammatory interleukin.

8. A pharmaceutical composition for use as a medicament in the treatment of cervical dysplasia for local external application in the region of the portio and cervical uteri, comprising at least the agent according to one or more of the preceding claims, in addition to galenic additives, which are suitable for external local application in the region of the portio and cervical uteri.

9. The pharmaceutical composition for use according to claim 8, comprising at least one further pharmaceutically active substance.

10. The pharmaceutical composition for use according to claim 8 or 9, wherein said composition is present as a suspension, gel, ointment, lotion or as a solution.

11. A medication for use in the treatment of cervical dysplasia for local external application in the region of the portio and cervical uteri, comprising a pharmaceutical composition according to claims 8 to 10.

## Revendications

1. Agent pouvant être utilisé comme médicament dans le traitement des dysplasies cervicales, comprenant un adénovirus recombiné, modifié génétiquement dans des cellules non infectées par le HPV, rendu non réplicatif par délétion de la région E, ledit agent étant destiné à une administration locale et externe dans la région du col de l'utérus et dans laquelle l'adénovirus à délétion de la région E1 comprend un ou plusieurs gènes de vaccination représentant des épitopes antigéniques de papillomavirus, dont l'expression permet de déclencher une réponse immunitaire cellulaire ou humorale contre des cellules infectées par le HPV ou exprimant des oncogènes de HPV.

2. Agent pouvant être utilisé selon la revendication 1, dans laquelle les gènes de vaccination induisent une réponse immunitaire contre les papillomavirus humains (HPV) 16, 18, 25, 31, 33, 35, 39, 45, 51, 52, 53, 56, 58, 59, 66, 68,73 ou 82, et plus préférentiellement contre le HPV 16 et le HPV 18.

3. Agent pouvant être utilisé selon la revendication 2, comprenant des gènes de vaccination fabriqués par fusion de fragments des oncogènes E6 et E7 de HPV provenant des HPV 16 et HPV 18 à *haut risque.*

4. Agent pouvant être utilisé selon la revendication 3, dans laquelle la séquence du gène de vaccination correspond à la SEQ ID No. 1.

5. Agent pouvant être utilisé selon la revendication 3, dans laquelle le gène de vaccination code pour la séquence protéique de la SEQ ID No. 2.

6. Agent pour une utilisation selon l'une quelconque des revendications précédentes, dans laquelle l'adénovirus à délétion de la région E1 comprend un ou plusieurs gènes immunomodulateurs, qui permettent de renforcer la réponse immunitaire contre les cellules infectées par le HPV.

7. Agent pouvant être utilisé selon la revendication 6, dans laquelle le gène immunomodulateur est celui d'une interleukine inflammatoire.

8. Composition pharmaceutique pouvant être utilisée comme médicament dans le traitement local et externe des dysplasies cervicales dans la région du col de l'utérus, comprenant au moins l'agent selon l'une quelconque des revendications précédentes, ainsi que des adjuvants galéniques appropriés à une administration locale et externe dans la région du col de l'utérus.

9. Composition pharmaceutique pouvant être utilisée selon la revendication 8, comprenant au moins une substance active pharmaceutique supplémentaire.

10. Composition pharmaceutique pouvant être utilisée selon la revendication 8 ou 9, dans laquelle celle-ci se présente sous la forme d'une suspension, d'un gel, d'une crème, d'une lotion ou d'une solution.

11. Médicament pouvant être utilisé dans le traitement local et externe des dysplasies cervicales dans la région du col de l'utérus, comprenant une composition pharmaceutique selon les revendications 8 à 10.


### SEQUENCE LISTING

<110> Charité - Universitätsmedizin Berlin
<120> Mittel zur lokalen Behandlung von Zervixdysplasien
<130> XI 197/11
<150> DE 10 2010 016 475.5
   <151> 2010-04-16
<160> 2
<170> PatentIn version 3.3
<210> 1
   <211> 1248
   <212> DNA
   <213> artificial, from Human papilloma virus (HPV)
<400> 1
   atggactcta cgcttcggtt gtgcgtacaa agcacacacg tagacattcg tactttggtc     60
   gaccaattaa atgacagctc agaggaggag gatgaaatag atggtccagc tggacaagca    120
   gaaccggaca gagcccatta caatattgta acctttgggc ccatgcatgg agatacacct    180
   acattgcatg aatatatgtt agatttgcaa ccagagacaa ctgatcaatt gaagtgtgaa    240
   gctagaattg agctagtagt agaaagctca gcagacgacc ttcgagcatt cagatctcaa    300
   ttaagcgact cagaggaaga aaacgatgaa atagatggag ttaatcatca acatttacca    360
   gcccgacgag ccgaaccaca acgtcacaca atgttggcta gcatgcatgg acctaaggca    420
   acattgcaag acattgtatt gcatttagag cctcaaaatg aaattccggt tgacggtacc    480
   aagcaaagac atctggacaa aaagcaaaga ttccataata taaggggtcg gtggaccggt    540
   cgatccggat taaagtttta ttctaaaatt agtgagtata gacattattg ttatagtttg    600
   tatggaacaa cattagaaca gcaatacaac aaaccgttgt gtgatttgtt aattaggcct    660
   aggaagcaac agttactgcg acgtgaggta tatgactttg cttttcggga tttatgcata    720
   gtatatagag atgggaatcc atatgctgta ccgcggccac aggagcgacc cagaaagtta    780
   ccacagttat gcacagagct gcaaacaact atacatgata taatattaga atcgcgaaat    840
   gaaaaacgac gattccacaa aatagctggg cactatagag gccagctcga gatagatttt    900
   tattctagaa ttagagaatt aagacattat tcagactctg tgtatggaga cacattagaa    960
   aaactaacta acactgggtt atacaattta ttaataagga ctagtaagac agtattggaa   1020
   cttacagagg tatttgaatt tgcattcaaa gatttatttg tggtgtatag agacagtata   1080
   ccgcatgctg cacacgtgcc aacacggcga ccctacaagc tacctgatct gtgcacggaa   1140
   ctgaacactt cactgcaaga catagaaata acccttaagc tgatcccacg tcactattgt   1200
   atactctata ttatactcta tgttatactc tgtaatccta ctcaataa                1248
<210> 2
   <211> 415
   <212> PRT
   <213> artificial, from Human papilloma virus (HPV)
<400> 2
   Met Asp Ser Thr Leu Arg Leu Cys Val Gln Ser Thr His Val Asp Ile
   1 5 10 15
   Arg Thr Leu Val Asp Gln Leu Asn Asp Ser Ser Glu Glu Glu Asp Glu
   20 25 30
   Ile Asp Gly Pro Ala Gly Gln Ala Glu Pro Asp Arg Ala His Tyr Asn
   35 40 45
   Ile Val Thr Phe Gly Pro Met His Gly Asp Thr Pro Thr Leu His Glu
   50 55 60
   Tyr Met Leu Asp Leu Gln Pro Glu Thr Thr Asp Gln Leu Lys Cys Glu
   65 70 75 80
   Ala Arg Ile Glu Leu Val Val Glu Ser Ser Ala Asp Asp Leu Arg Ala
   85 90 95
   Phe Arg Phe Gln Leu Ser Asp Ser Glu Glu Glu Asn Asp Glu Ile Asp
   100 105 110
   Gly Val Asn His Gln His Leu Pro Ala Arg Arg Ala Glu Pro Gln Arg
   115 120 125
   His Thr Met Leu Ala Ser Met His Gly Pro Lys Ala Thr Leu Gln Asp
   130 135 140
   Ile Val Leu His Leu Glu Pro Gln Asn Glu Ile Pro Val Asp Gly Thr
   145 150 155 160
   Lys Gln Arg His Leu Asp Lys Lys Gln Arg Phe His Asn Ile Arg Gly
   165 170 175
   Arg Trp Thr Gly Arg Ser Gly Leu Lys Phe Tyr Ser Lys Ile Ser Glu
   180 185 190
   Tyr Arg His Tyr Cys Tyr Ser Leu Tyr Gly Thr Thr Leu Glu Gln Gln
   195 200 205
   Tyr Asn Lys Pro Leu Cys Asp Leu Leu Ile Arg Pro Arg Lys Gln Gln
   210 215 220
   Leu Leu Arg Arg Glu Val Tyr Asp Phe Ala Phe Arg Asp Leu Cys Ile
   225 230 235 240
   Val Tyr Arg Asp Gly Asn Pro Tyr Ala Val Pro Arg Pro Gln Glu Arg
                   245                 250                 255
   Pro Arg Lys Leu Pro Gln Leu Cys Thr Glu Leu Gln Thr Thr Ile His
               260                 265                 270
   Asp Ile Ile Leu Glu Ser Arg Asn Glu Lys Arg Arg Phe His Lys Ile
           275                 280                 285
   Ala Gly His Tyr Arg Gly Gln Leu Glu Ile Asp Phe Tyr Ser Arg Ile
       290                 295                 300
   Arg Glu Leu Arg His Tyr Ser Asp Ser Val Tyr Gly Asp Thr Leu Glu
   305                 310                 315                 320
   Lys Leu Thr Asn Thr Gly Leu Tyr Asn Leu Leu Ile Arg Thr Ser Lys
                   325                 330                 335
   Thr Val Leu Glu Leu Thr Glu Val Phe Glu Phe Ala Phe Lys Asp Leu
               340                 345                 350
   Phe Val Val Tyr Arg Asp Ser Ile Pro His Ala Ala His Val Pro Thr
           355                 360                 365
   Arg Arg Pro Tyr Lys Leu Pro Asp Leu Cys Thr Glu Leu Asn Thr Ser
       370                 375                 380
   Leu Gln Asp Ile Glu Ile Thr Leu Lys Leu Ile Pro Arg His Tyr Cys
   385                 390                 395                 400
   Ile Leu Tyr Ile Ile Leu Tyr Val Ile Leu Cys Asn Pro Thr Gln
                   405                 410                 415